# EUROPEAN PATENT APPLICATION

(11) **EP 3 333 152 A1**
(43) Date of publication of application: **13.06.2018**
(21) Application number: 17207546.7
(22) Date of filing: 22.02.2017
(51) Int. Cl.: C07C 69/44, C07C 55/14, C07C 57/16

(54) **DEOXYDEHYDRATION OF SUGAR DERIVATIVES**

(30) Priority: 25.02.2016 US 201662300008 P; 22.03.2016 US 201662311488 P
(62) Divisional of application: 17757088.4
(71) Applicant: The Regents of The University of California Santa Cruz, Oakland, CA 94607 (US); BASF SE, 67056 Ludwigshafen am Rhein (DE)
(72) Inventor: Toste, Dean F., Piedmont, CA 94611 (US); Larson, Reed T., Jersey City, NJ 07310 (US); Bohn, Martin A., 67056 Ludwigshafen am Rhein (DE)
(74) Representative: Eisenführ Speiser

(57) **Abstract**

The disclosure provides methods for deoxydehydration of sugar-based derivatives using hydrogen gas as a reducing agent.

## Description

### CROSS REFERENCE TO RELATED APPLICATIONS

This application claims the benefit of U.S. Provisional Application No. 62/300,008, filed February 25, 2016, and Provisional Application No.62/311,488, filed March 22, 2016, the disclosures of which are incorporated herein by reference in their entirety.

### TECHNICAL FIELD

The disclosure provides methods for deoxydehydration of sugar-based derivatives using hydrogen gas as a reducing agent.

### BACKGROUND

Lignocellulosic biomass is the most abundant resource of organic carbon on Earth and is the only renewable resource that is cheap enough to replace fossil fuels and sustain energy demands in the transportation sector. Such biomass is composed of three major polymeric components: cellulose, hemicellulose, and lignin. Cellulose is crystalline in structure and is comprised of linear β-1,4 linked glucose units known as glucan. Hemicellulose is amorphous in structure and is often primarily comprised of polymeric chains of β-1,4 linked xylose units known as xylan, a major hemicellulose component in most hardwood species, agricultural residues, and herbaceous energy crops. Lignin is a crosslinked heterogeneous complex covalently bonded to hemicellulose involving polymers of phenyl propanol units called monolignols.

### SUMMARY

The disclosure provides a method for the deoxydehydration of vicinal diols allowing for access of deoxygenated analogues of sugar-based derivatives. The methods of the disclosure allow for the use of carboxylic acids and esters derived from sugars as substrates and hydrogen gas as a reducing agent.

In a particular embodiment, the disclosure provides a method for the deoxydehydration (DODH) of a sugar derivative comprising: (a) incubating a reaction mixture for a sufficient period of time to allow for formation of one or more deoxydehydrated products, wherein the reaction mixture comprises: (i) a reactant selected from the group consisting of an aldaric acid, an aldaric acid derivative, an aldonic acid, aldonic acid derivative, a sugar lactone, and a sugar lactone derivative; (ii) a catalyst selected from the group consisting of a vanadium-based catalyst, a molybdenum-based catalyst, a rhenium-based catalyst, and any combination thereof; (iii) a reducing agent comprising hydrogen gas; (iv) a solvent system; and (v) optionally an acid.

In another embodiment, the reaction is carried out by incubating the reaction mixture at a temperature greater than 20°C. In yet another embodiment, the reaction mixture is incubated at a temperature between 120°C to 300°C. In a further embodiment, the reaction is carried out for up to 72 hours. In yet a further embodiment, the reaction mixture is incubated at about 150°C for up to 4 hours.

In accordance with an important feature of the present invention, it was found that only vicinal diols in an α, β-position to an electron withdrawing group, such as a carbonyl group, undergo a DODH reaction. Examples of electron withdrawing groups are carboxylic acid, ester, and lactone.

In a certain embodiment, for a method disclosed herein, the catalyst can be regenerated and reused (*e.g*., step (a)) by exposing the catalyst to an oxidizing agent comprising oxygen gas. In a further embodiment, after performing step (a), the method further comprises: (b') adding to the reaction mixture a catalyst selected from the group consisting of a vanadium-based catalyst, a palladium-based catalyst, a platinum-based catalyst, a nickel-based catalyst, a molybdenum-based catalyst, a lithium-based catalyst, an aluminum based-catalyst, an iron-based catalyst, an iridium-based catalyst, a rhodium-based catalyst, a rhenium-based catalyst, and any combination thereof; and subsequently or simultaneously increasing the pressure of the hydrogen gas up to 300 psi and heating the reaction mixture at a temperature between 120°C to 160°C.

In a particular embodiment, a method disclosed herein can be repeated one or more times. In another embodiment, a method disclosed herein further comprises separating the product from any remaining reactant and reaction intermediates. In yet a further embodiment, a method disclosed herein is performed using a one pot synthesis strategy.

In a certain embodiment, a method disclosed herein produces one or more reduced product(s) comprising at least one reduced product that comprises a structure selected from the group consisting of formula I, formula II, formula III, and formula IV: 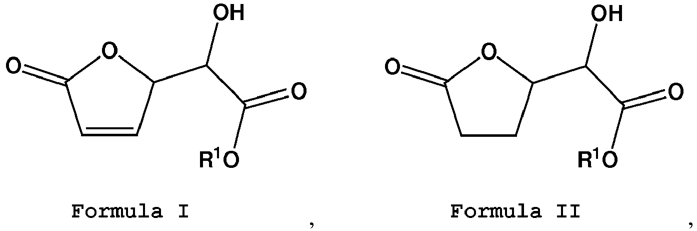 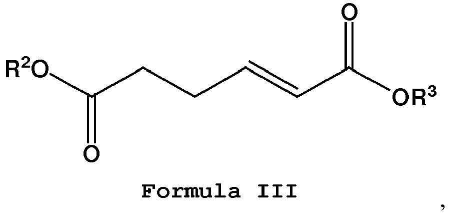 and 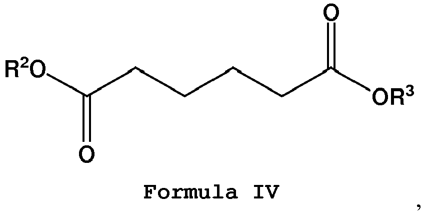 wherein, R¹, R², and R³ are each independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl. In a further embodiment, a reduced product of formula IV is produced from the reduced products of formula I, or from formula II that is produced from the compound of formula I, or from formula III that is produced from formula II that is produced from formula I.

In a certain embodiment, the disclosure provides for a method for the deoxydehydration (DODH) of a sugar derivative comprising: (a) incubating a reaction mixture for a sufficient period of time to allow for formation of one or more deoxydehydrated products, wherein the reaction mixture comprises: (i) a reactant selected from the group consisting of an aldaric acid, an aldaric acid derivative, an aldonic acid, aldonic acid derivative, a sugar lactone, and a sugar lactone derivative; (ii) a catalyst selected from the group consisting of a vanadium-based catalyst, a molybdenum-based catalyst, a rhenium-based catalyst and any combination thereof; (iii) a reducing agent comprising hydrogen gas; (iv) a solvent system; and (v) optionally an acid; (b) adding to the reaction mixture one or more catalysts suitable for the hydrogenation of an alkene and/or increasing the pressure of hydrogen gas up to 300 psi; followed by (c) repeating step (a); followed by (d) repeating step (b); and (e) optionally repeating steps (a) and (b) until the majority of the sugar derivatives has been converted to a reduced product having the structure of formula IV: 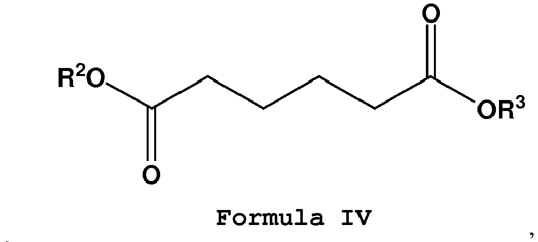 wherein, R² and R³ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl. In a further embodiment, steps (a), (b), (c), (d) and/or (e) are carried out at a temperature from 20°C to 300°C.

In a particular embodiment, a method disclosed herein comprises an aldaric acid reactant and the deoxydehydrated product is an unsaturated dicarboxylic acid compound. In an alternate embodiment, a method disclosed herein comprises a glucaric acid reactant and the one or more deoxydehydrated products is adipic acid.

In yet another embodiment, a method disclosed herein uses a rhenium-based catalyst. Examples of rhenium-based catalyst include, but are not limited to, HReO₄, KReO₄, NH₄ReO₄, ReO₂, ReIO₂(Ph₃P)₂, ReCl₃O(Ph₃P)₂, CH₃ReO₃ (MTO), and ReCl₃. In a certain embodiment, a method of the disclosure uses MTO or HReO₄ catalyst. In an alternate embodiment, a method disclosed herein uses a vanadium-based catalyst. Examples of vanadium-based catalysts include, but are not limited to, NBu₄VO₃, NBu₄VO₂(CA)₂, HC(PZ)VO₂BF₄, TpaVO₂PF₆, NaVO₂(acac)₂, and Bu₄N(dipic)VO₂. In yet another embodiment, a method disclosed herein uses molybdenum-based catalysts. Examples of molybdenum-based catalysts include, but are not limited to, MoO₃, Mo(CO)₆, Mo(CO)₄(bipy), MOO₂Cl₂(bipy), MoO₂Br₂(bipy), MoO₂(CH₃)₂(bipy),(NH₄)₆Mo₇O₂₄·4H₂O, and H₃PMo₁₂O₄₀.

In a further embodiment, a method disclosed herein comprises palladium on carbon (Pd/C), sodium sulfite, triphenylphospine, and/or secondary alcohols. In yet a further embodiment, a method disclosed herein comprises Pd/C.

Surprisingly, it was found that the addition of a second component to the catalyst system, e.g., Pd/C, improved the DODH capabilities of the catalyst system. For example: the addition of Pd/C increases the reaction speed (4 hours to 0.75 hours) and the yield to 90% from 55%. Thus, in a further embodiment, the method disclosed herein additionally comprises Pd/C.

In a certain embodiment, a method disclosed herein comprises a solvent system which comprises ones or more polar solvents. Examples of polar solvents include but are not limited to water, methanol, ethanol, *n*-propanol, *n*-butanol, isopropanol, acetic acid, and formic acid. In another embodiment, a method disclosed herein comprises a solvent system which comprises ethanol and/or methanol.

In a particular embodiment, the disclosure also provides a method to produce (C₄-C₇)-linear saturated carboxylic acids from polysaccharides and/or disaccharides comprising: (A) polysaccharides and/or disaccharides with enzymes to hydrolyze the polysaccharides and/or disaccharides into simple sugars; (B) oxidizing the simple sugars to form aldonic acids or aldaric acids; and (C) deoxydehydrating the aldonic acids or aldaric acids using any one of the preceding methods to produce (C₄-C₇)-linear saturated carboxylic acids; or optionally (B') derivatize the aldonic acid or aldaric acid of step (B), and (C') deoxydehydrating the aldonic acid derivatives or aldaric acid derivatives using any one of the preceding methods to produce (C₄-C₇) linear saturated carboxylic acids and/or (C₄-C₇)-linear saturated carboxylic acid derivatives, which may be hydrolyzed to the (C₄-C₇)-linear saturated carboxylic acids.

In yet another embodiment, the disclosure provides a method to produce (C₄-C₇)-linear saturated carboxylic acids from a lignocellulosic biomass comprising: (A) pretreating the lignocellulosic biomass with one or more physical processes, one or more chemical processes, and/or one or more biological agent(s) or any combination thereof to generate solubilized lignocellulosic polymers; (B) hydrolyzing the lignocellulosic polymers using enzymes and/or chemical treatment to obtain simple sugars; (C) oxidizing the simple sugars to form aldaric acids or aldonic acids; and (D) deoxydehydrating the aldaric acids or aldonic acids using any one of the disclosed methods to produce (C₄-C₇)-linear saturated carboxylic acids; or optionally (C') derivatize the aldonic acid or aldaric acid of step (C); and (D') deoxydehydrating the aldonic acid derivatives or aldaric acid derivatives using any one of the preceding methods to produce (C₄-C₇)-linear saturated carboxylic acids and/or (C₄-C₇)-linear saturated carboxylic acids derivatives, which may be hydrolyzed to the (C₄-C₇)-linear saturated carboxylic acids. In yet another embodiment, the lignocellulosic biomass is pretreated with one or more physical processes and/or with acid; the lignocellulosic polymers are hydrolyzed by enzymatic action; and/or the simple sugars are oxidized by treating with nitric acid. In a further embodiment, the aldaric acids using in the reaction mixture comprises glucaric acid, and wherein the (C₄-C₇)-linear saturated carboxylic acid derivative comprises adipic acid esters and wherein the (C₄-C₇)-linear saturated carboxylic acid comprises adipic acid.

In a particular embodiment, a method disclosed herein comprises a reaction mixture that comprises an aldaric acid derivative reactant having the structure of: 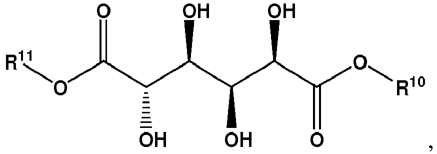 wherein, R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl, wherein at least one of R¹⁰ or R¹¹ is not H. In a further embodiment, the reaction mixture which comprises the aldaric acid derivative is incubated at a temperature greater than 20°C. In yet a further embodiment, the reaction mixture which comprises the aldaric acid derivative is incubated at a temperature between 120°C to 300°C. In another embodiment, the reaction mixture which comprises the aldaric acid derivative is incubated for up to 72 hours. In yet another embodiment, the reaction mixture which comprises the aldaric acid derivative is incubated at about 150°C for up to 4 hours. In a further embodiment, the reaction mixture which comprises the aldaric acid derivative comprises a rhenium-based catalyst (*e.g*., MTO). In yet a further embodiment, the reaction mixture which comprises the aldaric acid derivative comprises a solvent system which comprises an alcohol (*e.g*., ethanol). In another embodiment, the reaction mixture which comprises the aldaric acid derivative comprises palladium on carbon (Pd/C).

In yet a further embodiment, the reaction mixture which comprises the aldaric acid derivative produces a deoxydehydrated product which comprises a lactone derivative having a structure of: 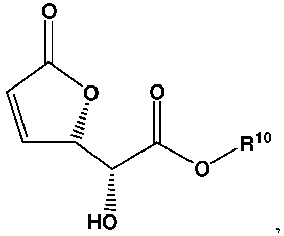 wherein, R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl. In yet a further embodiment, a method disclosed herein comprises a reaction mixture that comprises the lactone derivative with one or more reducing agents comprising hydrogen gas. In another embodiment, the hydrogen gas is used at a pressure up to 300 psi. In another embodiment, a reaction mixture that comprises the lactone derivative is incubated at a temperature greater than 20°C in a solvent system comprising a catalyst suitable for the hydrogenation of an alkene. In yet a further embodiment, a reaction mixture that comprises the lactone derivative comprises a solvent system comprising an alcohol (*e.g*., ethanol). In yet another embodiment, a reaction mixture that comprises the lactone derivative further comprises a catalyst comprising Pd/C.

In yet a further embodiment, a reaction mixture that comprises the lactone derivative produces a reduced product having a structure of: 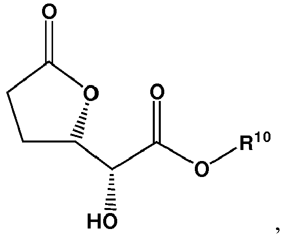 wherein, R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.

In a certain embodiment, a method of deoxydehydrating a reduced lactone product having the structure of 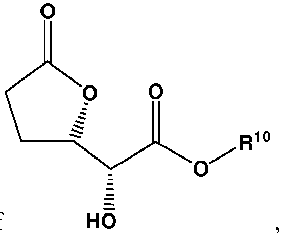 comprises incubating a reaction mixture comprising the reduced product for a sufficient period of time to allow for formation of a deoxydehydrated product, wherein the reaction mixture comprises: a catalyst selected from the group consisting of a vanadium-based catalyst, a molybdenum-based catalyst, a rhenium-based catalyst and any combination thereof; a reducing agent comprising hydrogen gas; a solvent system; and optionally an acid, wherein R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl. In another embodiment, a reaction mixture comprising the reduced lactone product is incubated at a temperature greater than 20°C. In yet another embodiment, a reaction mixture comprising the reduced lactone product is incubated at a temperature between 120°C to 300°C. In a further embodiment, a reaction mixture comprising the reduced lactone product is incubated for up to 72 hours. In yet a further embodiment, a reaction mixture comprising the reduced lactone product is incubated at about 150°C for up to 4 hours. In a certain embodiment, a reaction mixture comprising the reduced lactone product comprises a rhenium-based catalyst. Examples of rhenium-based catalysts include, but are not limited to, HReO₄, KReO₄, NH₄ReO₄, ReO₂, ReIO₂(Ph₃P)₂, ReCl₃O(Ph₃P)₂, CH₃ReO₃ (MTO), and ReCl₃. In a particular embodiment, a reaction mixture comprising the reduced lactone product comprises MTO. In another embodiment, a reaction mixture comprising the reduced lactone product comprises an alcohol (*e.g*., ethanol). In yet another embodiment, a reaction mixture comprising the reduced lactone product further comprises Pd/C. In a further embodiment, a reaction mixture comprising the reduced lactone product produces hex-2-enedioic acid diethyl ester.

In a particular embodiment, the disclosure also provides a method of reacting hex-2-enedioic acid diethyl ester with one or more reducing agents comprising hydrogen gas. In a further embodiment, the hydrogen gas is used at a pressure of up to 300 psi. In yet a further embodiment, the hex-2-enedioic acid diethyl ester is reduced at a temperature greater than 20°C in a solvent system comprising a catalyst suitable for the hydrogenation of an alkene. In another embodiment, the solvent system comprises an alcohol (*e.g*., ethanol). In yet another embodiment, the catalyst comprises Pd/C. In a further embodiment, the method of reducing hex-2-enedioic acid diethyl ester produces diethyl adipate.

In a certain embodiment, the disclosure also provides a method for the deoxydehydration (DODH) of a sugar derivative, comprising:(a) incubating a reaction mixture at 220 to 295°C for a sufficient period of time to allow for formation of one or more deoxydehydrated products, wherein the reaction mixture comprises a reactant having the structure of 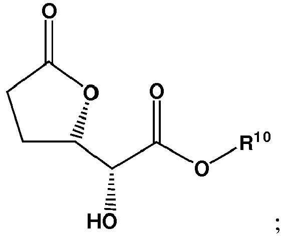 catalysts comprising (NH₄)₆Mo₇O₂₄ and Pd/C; a reducing agent comprising hydrogen gas; a solvent system comprising ethanol, wherein, R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.

In a certain embodiment, a method disclosed herein comprises a reaction mixture which comprises a sugar lactone derivative having the structure of Formula V or Formula V(a): 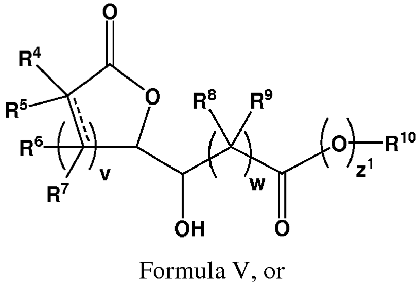 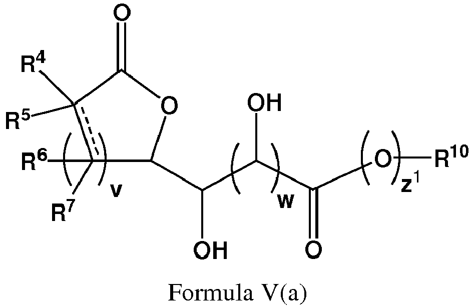 wherein, v is an integer selected from the group consisting of 1, 2, 3, 4, and 5; w is an integer selected from the group consisting of 0, 1, 2, 3, 4, 5 and 6; z¹ is an integer selected from 0 or 1; R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl. In a further embodiment, the reaction mixture comprising the lactone of Formula V or Formula V(a) is incubated at a temperature between 120°C to 300°C. In yet a further embodiment, the reaction mixture comprising the lactone of Formula V or Formula V(a) is incubated for up to 72 hours. In another embodiment, the reaction mixture comprising the lactone of Formula V of Formula V(a) is incubated at about 150°C for up to 4 hours. In yet another embodiment, the reaction mixture comprising the lactone of Formula V of Formula V(a) comprises a rhenium-based catalyst (*e.g*., MTO). In yet another embodiment, the reaction mixture comprising the lactone of Formula V of Formula V(a) comprises a solvent system comprising an alcohol (*e.g*., ethanol). In a certain embodiment, the reaction mixture comprising the lactone of Formula V of Formula V(a) further comprises palladium on carbon (Pd/C).

In another embodiment, the reaction mixture comprising the lactone of Formula V or Formula V(a) produces a structure of Formula VI or Formula VI(a): 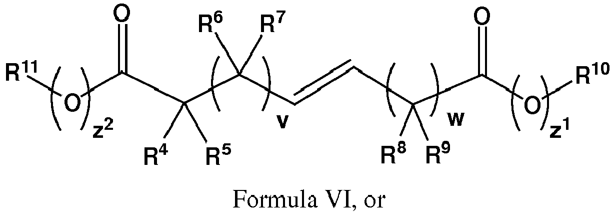 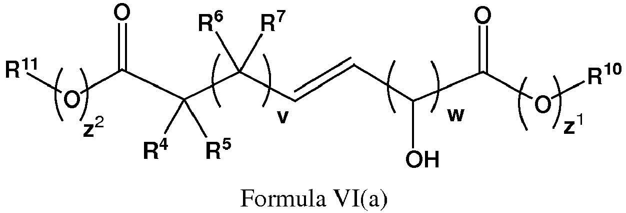 wherein, v is an integer selected from the group consisting of 1, 2, 3, 4, and 5; w is an integer selected from the group consisting of 0, 1, 2, 3, 4, 5 and 6; z¹ and z² are independently selected integers from 0 or 1; R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.

In yet another embodiment, the method further comprises reducing the deoxydehydrated product of Formula VI or Formula VI(a) using one or more reducing agents comprising hydrogen gas. In a further embodiment, the hydrogen gas is used at pressure of up to 300 psi. In yet a further embodiment, the deoxydehydrated product of Formula VI or Formula VI(a) is reduced at a temperature greater than 20°C in a solvent system comprising a catalyst suitable for hydrogenating an alkene. In another embodiment, the solvent system comprises an alcohol (*e.g*., ethanol). In yet another embodiment, the catalyst comprises Pd/C. In a particular embodiment, the reduction of the deoxydehydrated product of Formula VI or Formula VI(a) produces a reduced product having a structure of Formula VII: 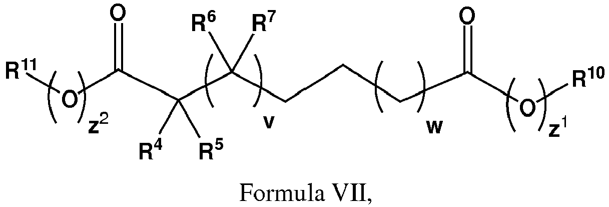 wherein, v is an integer selected from the group consisting of 1, 2, 3, 4, and 5; w is an integer selected from the group consisting of 0, 1, 2, 3, 4, 5 and 6; z¹ and z² are integers independently selected from 0 or 1; R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.

### DESCRIPTION OF DRAWINGS

**Figure 1** provides an embodiment of a process to produce C₃-C₇ commodities from lignocellulosic biomass using the DODH methods disclosed herein.
**Figure 2** illustrates the traditional scheme to convert vicinal diols to olefin products using high valent oxo-rhenium catalysts with various reducing agents. Examples of Re catalysts include: HReO₄, methyl trioxorhenium (MTO), NH₄ReO₄, CpReO3, TpReO₃, and ReOX/C. Examples of Reductants include: PPH₃, Na₂SO₃, H₂, and alcohols.
**Figure 3A-B** provides examples of schemes to produce deoxydehydration products from sugar based substrates. (**A**) Deoxydehydration of sorbitol produces hexatriene; and (**B**) Deoxydehydration of glucaric acid produces muconic acid, which can be further reduced to adipic acid.
**Figure 4** presents reaction conditions for the conversion of ribonolactone into a derivative of levulinic acid. Also shown, is the failure to convert xylonolactone into a similar derivative of levulinic acid using the same reaction conditions.
**Figure 5** presents reaction conditions for the conversion of glucaro-6,3-lactone into a DODH adduct.
**Figure 6A-B** presents the yields of a DODH lactone adduct from diethyl glucarate using the specified reaction conditions. (**A**) Reaction performed using H₂ as a reducing agent; and (**B**) Reaction performed using H₂ as a reducing agent and the catalyst palladium on carbon (Pd/C).
**Figure 7** presents a scheme showing the production of diethyl adipate from diethyl glucarate through four reactions using hydrogen gas as a reducing agent. Yields are further increased with the use of a catalyst suitable for the hydrogenation of an alkene, such as Pd/C.
**Figure 8** presents a 'one pot' synthesis strategy showing the production of diethyl adipate from diethyl glucarate in good yields by only using hydrogen gas as the reducing agent and by adding additional catalyst after the initial reaction step.
**Figure 9** presents reaction conditions for the production of diethyl adipate from diethyl glucarate by just changing H₂ pressure after the initial reaction step (no additional catalyst was added).

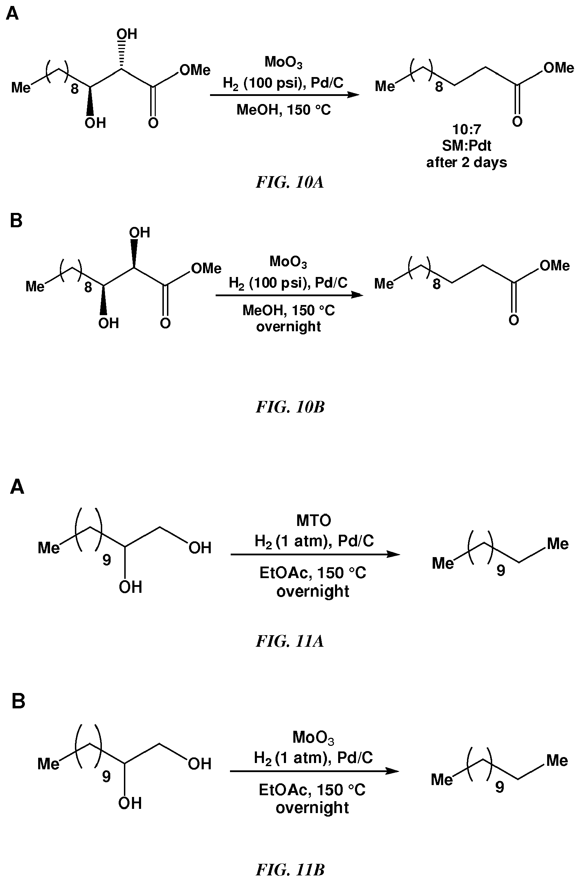

**Figure 10A-B** presents reaction conditions for the production of alkyl esters from α,β hydroxyester substrates using a molybdenum-based catalyst. (**A**) Conversion of a *trans-*α,β hydroxyester to an alkyl ester product; and (**B**) conversion of a *cis-*α*,*β hydroxyester to an alkyl ester product. 'SM' refers to starting material, and 'Pdt' refers to product.
**Figure 11A-B** presents reaction conditions for the production of alkanes from terminal diol substrates using rhenium or molybdenum based catalysts. (**A**) Conversion of a terminal diol to an alkane using a rhenium catalyst; and **(B)** Conversion of a terminal diol to an alkane using a molybdenum-based catalyst.

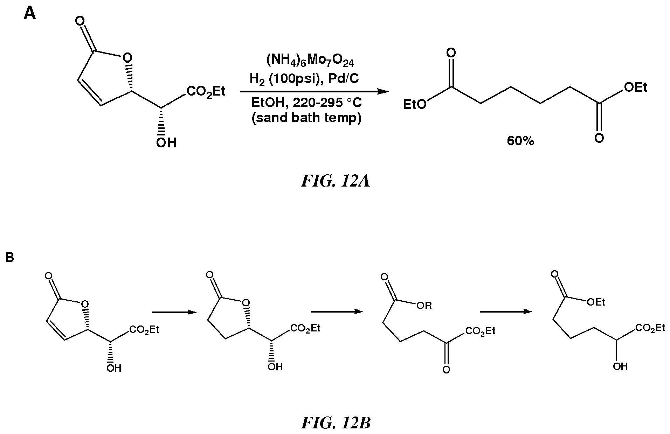

**Figure 12A-B** presents reaction conditions for the formation of diethyl adipate from a glucaric acid derivative substrate and using a molybdenum-based catalyst. **(A)** Reaction conditions for diethyl adipate formation from a lactone in 60% yield. (**B**) Proposed mechanism for diethyl adipate formation, based upon analysis of the reaction at lower temperatures that showed significant formation of the mono hydroxy-product. It is hypothesized that the reaction sequence comprises Pd-mediated hydrogenation, elimination, and ketone reduction. A similar elimination chemistry was also seen with using a rhenium-based catalyst (perrhenic acid).

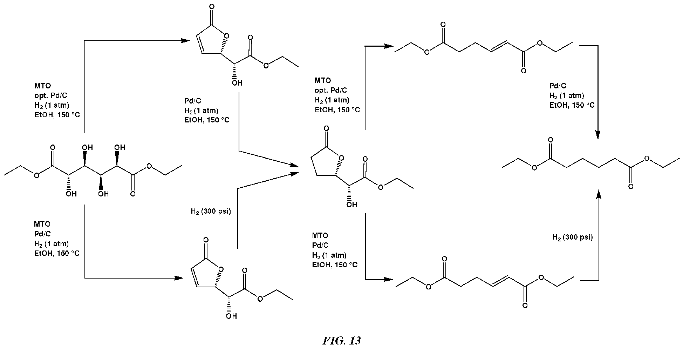

**Figure 13** presents a general overall scheme that allows for the formation of a hexanedioic acid diethyl ester end product from an aldaric acid derivative reactant by using DODH and reduction methods of the disclosure.

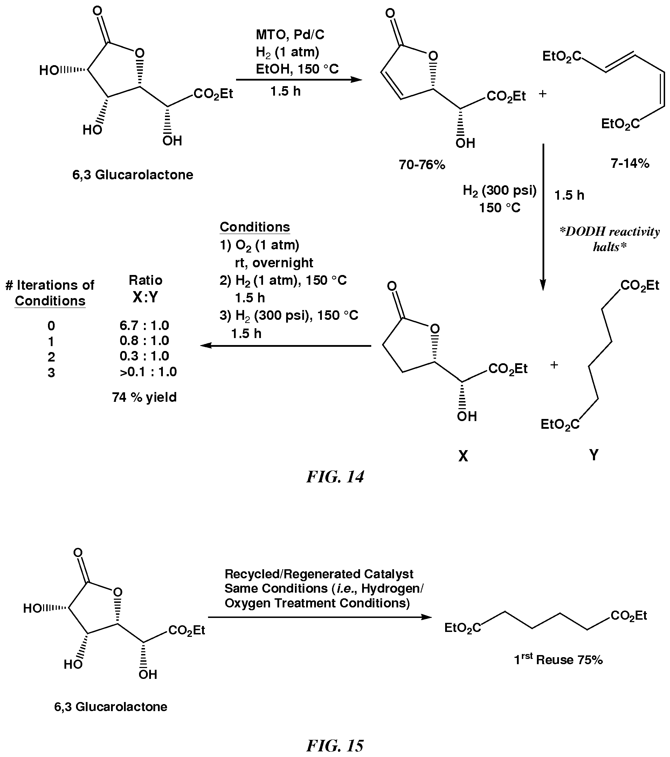

**Figure 14** presents a one pot conversion of 6,3 glucarolactone to diethyl adipate. As shown, the DODH catalysis is regenerated by exposure to oxygen without the need to add more MTO.
**Figure 15** shows that the catalyst can be reused with fresh starting material. The heterogeneous catalyst is centrifuged from the reaction mixture, rinsed with EtOH, and stirred in a solution of EtOH under 1 atm O₂ overnight. This "regenerated" catalyst is now reusable for DODH.

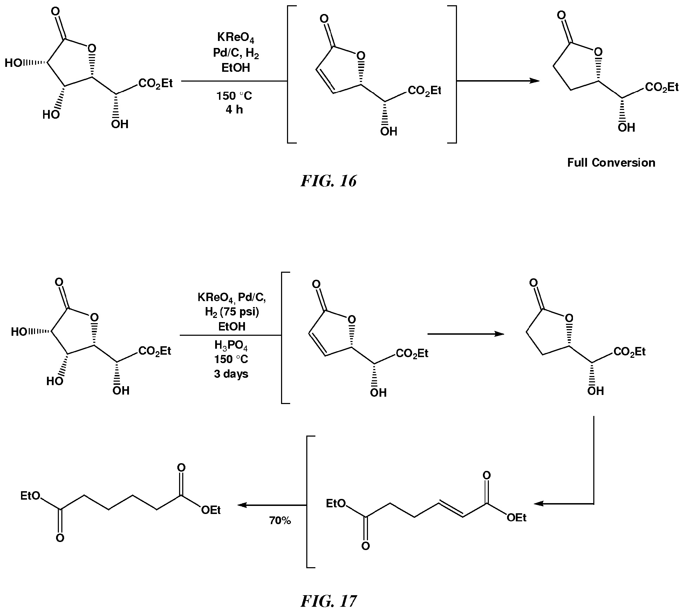

**Figure 16** demonstrates that full conversion of 6,3 glucarolactone can be brought about by using KReO₄ and palladium. Palladium mediated DODH with KReO₄ behaves much differently than MTO. The reaction does not stop at the unsaturated lactone, but it proceeds to the saturated lactone within 4 hours.
**Figure 17** demonstrates the effect of acidic additives on the KReO₄ system. Phosphoric acid allows for full conversion to diethyl adipate, with reduced sensitivity to H₂ pressure. This system does not require differential H₂ pressures or oxygen treatments.

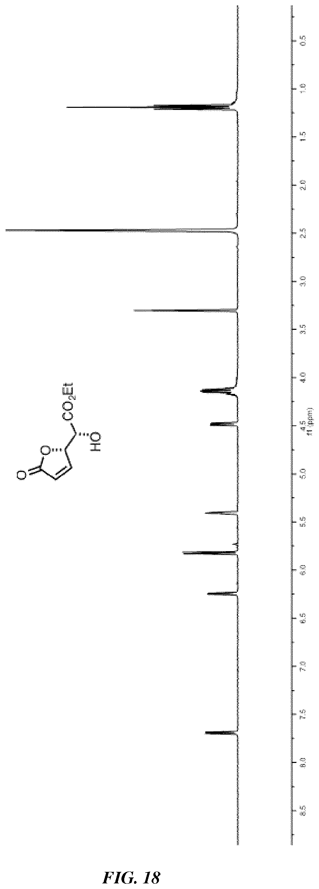

**Figure 18** presents the ¹H NMR of hydroxyl-(5-oxo-2,5-dihydro-furan-2-yl)-acetic acid ethyl ester.

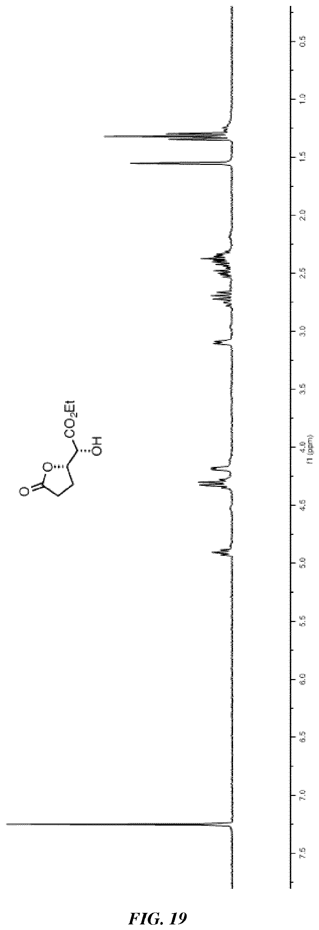

**Figure 19** presents the ¹H NMR of hydroxyl-(5-oxo-tetrahydro-furan-2-yl)acetic acid ethyl ester (*i.e*., DODH lactone adduct).

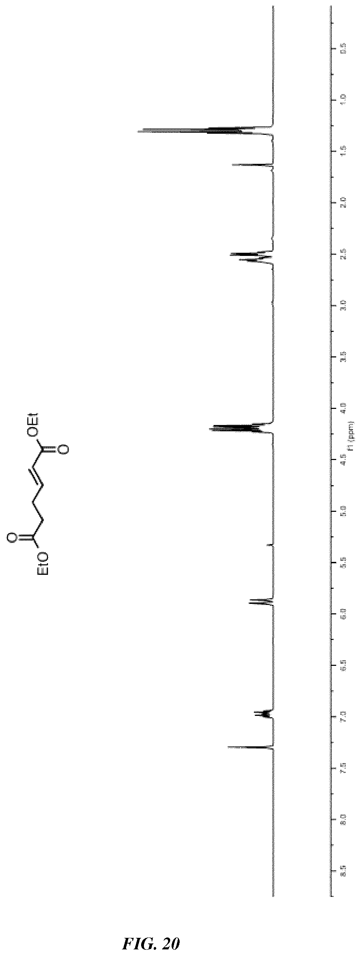

**Figure 20** presents the ¹H NMR of diethyl adipate.

### DETAILED DESCRIPTION

As used herein and in the appended claims, the singular forms "a," "an," and "the" include plural referents unless the context clearly dictates otherwise. Thus, for example, reference to "a catalyst" includes a plurality of such catalysts and reference to "the reducing agent" includes reference to one or more reducing agents or equivalents thereof known to those skilled in the art, and so forth.

Unless defined otherwise, all technical and scientific terms used herein have the same meaning as commonly understood by one of ordinary skill in the art to which this disclosure belongs. Although many methods and reagents similar to or equivalent to those described herein can be used in the practice of the disclosed methods and compositions, the exemplary methods and materials are now described.

All publications mentioned herein are incorporated herein by reference in their entirety for the purposes of describing and disclosing methodologies that might be used in connection with the description herein. Moreover, with respect to any term that is presented in the publications that is similar to, or identical with, a term that has been expressly defined in this disclosure, the definition of the term as expressly provided in this disclosure will control in all respects.

As used herein, the term "alkyl" refers to straight chain and branched saturated Cₙ₋ₚ hydrocarbon groups. Nonlimiting examples of alkyl groups include methyl, ethyl, and straight chain and branched propyl, butyl, pentyl, hexyl, heptyl, octyl, nonyl, and decyl groups. The term Cₙ means the alkyl group has "n" carbon atoms. The term Cₙ₋ₚ means that the alkyl group contains "n" to "p" carbon atoms. The term "alkylene" refers to an alkyl group having a substituent. An alkyl, e.g., methyl, or alkylene, e.g., -CH₂-, group can be unsubstituted or substituted with halo, trifluoromethyl, trifluoromethoxy, and alkoxy, for example.

As used herein, the terms "alkenyl" and "alkynyl" refer to unsaturated aliphatic groups analogous in length and optional substitution to the alkyls described above, but that contain at least one double or triple bond, respectively.

As used herein, the terms "heteroalkyl", "heteroalkenyl", and "heteroalkynyl" refer to an alkyl, alkenyl, or alkynyl group as defined above, wherein one to four carbon atoms are replaced by an oxygen, nitrogen, or sulfur atom, optionally substituted as described for an alkyl group.

As used herein, the term "cycloalkyl" and "cycloalkenyl" mean a monocyclic or bicyclic aliphatic ring system containing three to ten carbon atoms. A cycloalkenyl group contains at least one carbon-carbon double bond. The terms "heterocycloalkyl" and "heterocyclo" mean a monocyclic or bicyclic ring system containing three to ten total atoms and at least one nitrogen, oxygen, or sulfur atom in the ring system.

These ring systems are optionally substituted as described above for an alkyl group.

As used herein, the term "aryl" refers to a monocyclic or polycyclic aromatic group, preferably a monocyclic or bicyclic aromatic group, e.g., phenyl or naphthyl. Unless otherwise indicated, an aryl group can be unsubstituted or substituted with one or more, and in particular one to four, groups independently selected from, for example, halo, alkyl, -OCF₃, -CF₃, alkoxyl, aryl, and heteroaryl.

As used herein, the term "heteroaryl" refers to a monocyclic or bicyclic ring system containing one or two aromatic rings and containing at least one and up to four nitrogen and/or oxygen and/or sulfur atom in an aromatic ring. Unless otherwise indicated, a heteroaryl group can be unsubstituted or substituted with one or more, and in particular one to four, substitutents selected from, for example, halo, alkyl, -OCF₃, -CF₃, alkoxy, aryl, and heteroaryl.

As used herein, the term "halo" is defined as encompassing fluoro, chloro, bromo, and iodo.

The term "hydroxy" is defined as -OH.

The term "alkoxy" is defined as -OR, wherein R is alkyl.

The term "alkenoxy" is defined as -OR, wherein R is alkenyl.

The term "amino" is defined as -NH₂ and the term "alkylamino" is defined as - NR₂, wherein at least one R is alkyl and the second R is alkyl or hydrogen.

The term "nitro" is defined as -NO₂.

The term "cyano" is defined as -CN.

The term "trifluoromethyl" is defined as -CF₃.

The term "trifluoromethoxy" is defined as -OCF₃.

The term "thiol" is defined as -SR, wherein R is defined as alkyl.

The term "ester" is defined as -C(=O)OR, wherein R is alkyl or aryl.

As used herein, a "sugar compound" refers to sweet, short-chain, soluble carbohydrate comprised of hydrogen, oxygen and carbon atoms. A "sugar compound" will typically have a chemical formula of Cₓ(H₂O)ₓ, where x is an integer from 3 to 7. Specific examples of sugar compounds include erythrose, threose, erythrulose, arabinose, lyxose, ribose, xylose, ribulose, xylulose, allose, altrose, glucose, mannose, gulose, idose, galactose, talose, psicose, fructose, sorbose, tagatose, sedoheptulose, and mannoheptulose.

As used herein, the term "sugar derivative" refers to a sugar compound in which one or more functional groups of the sugar compound have been substituted, removed or modified. Examples of "sugar derivatives" would include, but are not limited to, aldaric acids, aldonic acids, and sugar lactones, or a derivative of any of the foregoing. In a particular embodiment, a "sugar derivative" refers to an ester or a carboxylic acid derivative of a sugar compound (*e.g*., a hydroxyl group and/or a ketone/aldehyde group of a sugar compound has been replaced with an ester or carboxylic group). In a further embodiment, a "sugar derivative" comprises 4 to 7 carbon atoms.

As used herein, an "aldaric acid" refers to a compound in which a terminal hydroxyl and aldehyde group of a sugar compound has been replaced with a carboxylic acid group Generally, an "aldaric acid" is characterized by the formula HOOC-(CHOH)ₙ-COOH. Examples of aldaric acids include, but are not limited to, tartaric acid, arabinaric acid, ribaric acid, xylaric acid, allaric acid, altraric acid, glucaric acid, talaric acid.

As used herein, an "aldaric acid derivative" refers to an aldaric acid compound in which one or more functional groups has been substituted, removed, or modified. For example, an "aldaric acid derivative" could include an aldaric acid compound where one or more of the terminal carboxylic acid groups are replaced with ester groups.

As used herein, an "aldonic acid" refers to compound in which an aldehyde or hydroxyl group of a sugar compound has been replaced with a carboxylic acid group. Examples of aldonic acids include, but are not limited to, arabinonic acid, ribonic acid, glyceric acid, gluconic acid, galacturonic acid, glucoronic acid, iduronic acid, threonic acid, and xylonic acid.

As used herein, an "aldonic acid derivative" refers to an aldaric acid compound in which a hydroxyl and/or a carboxylic acid group has been replaced or substituted with a different group. For example, an "aldonic acid derivative" could include an aldonic acid compound where a terminal carboxylic acid group was replaced with an ester.

As used herein, a "sugar lactone" refers to a cyclic ester compound that has formed from the dehydration of a sugar compound.

As used herein, a "sugar lactone derivative" refers to a sugar lactone that is derived from aldonic acid, aldonic acid derivative, aldaric acid, and an aldaric acid derivative. In a particular embodiment, a sugar lactone derivative comprises the structure of Formula V: 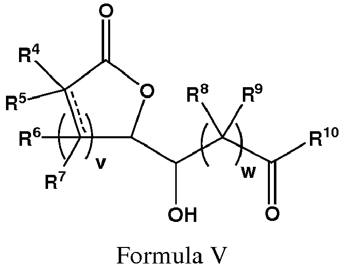 wherein,
v is an integer selected from the group consisting of 0, 1, 2, 3, 4, and 5;
w is an integer selected from the group consisting of 0, 1, 2, 3, 4, 5 and 6;
R⁴, R⁵, R⁶, R⁷, R⁸, and R⁹ are each independently an H, hydroxyl, halo, ester, ether, sulfide, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl; and
R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl; wherein the dash line indicates that the bond may be a single covalent bond or double covalent bond, and wherein if the bond is double covalent bond then R⁴ and R⁶ are absent.

In an alternate embodiment, the disclosure provides for a sugar lactone derivative comprising the structure of Formula V(a): 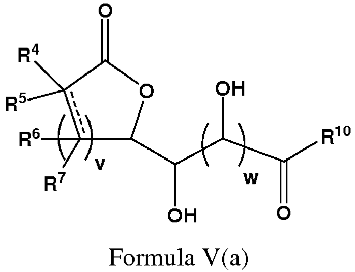 wherein,
v is an integer selected from the group consisting of 0, 1, 2, 3, 4, and 5;
w is an integer selected from the group consisting of 0, 1, 2, 3, 4, 5 and 6;
R⁴, R⁵, R⁶, R⁷, R⁸, and R⁹ are each independently an H, hydroxyl, halo, ester, ether, sulfide, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl; and
R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl; wherein the dash line indicates that the bond may be a single covalent bond or double covalent bond, and wherein if the bond is double covalent bond then R⁴ and R⁶ are absent. In a particular embodiment, the lactone ring of Formula V and Formula V(a) does not contain a doubly covalent carbon to carbon bond.

Lignocellulosic biomass is the most abundant resource of organic carbon on Earth and is a renewable resource that can economically replace fossil fuels for production of liquid fuels and sustain future energy demands in the transportation sector. Additionally, conversion of the lignocellulosic biomass to industrially desirable compounds, such as adipic acid, would also provide a great benefit. A feasible conversion strategy requires efficiently overcoming the recalcitrance of lignocellulose to maximize the yield of reactive sugar intermediates and their derivatives that are suitable for transformation to final products by targeted conversion technologies. The chemical transformation of lignocellulosic biomass provides an intriguing route to access C₃-C₆ commodities (*e.g*., malonic acid, succinic acid, glutaric acid, and adipic acid).

Cellulosic and lignocellulosic biomass residues and wastes, such as agricultural residues, wood, forestry wastes, sludge from paper manufacture, and municipal and industrial solid wastes, provide a potentially large renewable feedstock for the production of chemicals, plastics, fuels, and feeds. Cellulosic and lignocellulosic biomass residues and wastes, composed of carbohydrate polymers comprising cellulose, hemicellulose, and lignin can be generally treated by a variety of chemical, mechanical, and enzymatic means to release primarily hexose and pentose sugars, which are typically fermented to useful products including ethanol or dehydrated by acids to furfural, 5-HMf, and levulinic acid, which can then be catalytically upgraded to gasoline, diesel, and jet range fuels.

Pretreatment methods are used to make the carbohydrate polymers of cellulosic and lignocellulosic materials more readily available to saccharification enzymes or acid catalysts. Standard pretreatment methods have historically utilized primarily strong acids at high temperatures; however due to high energy costs, high equipment costs, high pretreatment catalyst recovery costs and incompatibility with saccharification enzymes, alternative methods are being developed, such as enzymatic pretreatment, or the use of acid or base at milder temperatures where decreased hydrolysis of biomass carbohydrate polymers occurs during pretreatment, requiring improved enzyme systems to saccharify both cellulose and hemicellulose. Additionally, carbohydrate polymers of cellulosic and lignocellulosic materials can be accessed by using one or more physical approaches, including, milling, chipping, grinding, pyrolysis, extrusion, explosion (*e.g.*, steam explosion, ammonia fiber explosion, carbon dioxide explosion) and irradiation (*e.g*., gamma rays, electron beam, ultrasounds, microwaves). Chemical pretreatment methods for lignocellulosic material, include but are not limited to, ozonolysis, acid hydrolysis, alkaline hydrolysis, oxidative delignification, and organosolv process. Additionally, pulsed electric-field pretreatment may also be employed. It should be further understood that the pretreatment of cellulosic and lignocellulosic materials may be accomplished by using any of the foregoing processes alone or alternatively in combination, *e.g*., pretreating the lignocellulosic materials with steam explosion, acid hydrolysis, and enzymatic treatment.

Glucaric acid is a member of a larger group of compounds known as sugar acids, and more specifically, aldaric acids. Glucaric acid has garnered attention because it was identified as one of the top 12 renewable building block chemicals by a 2004 US Department of Energy (DoE) report: *Top value added chemicals from biomass.* It can be prepared in one step from abundant and inexpensive glucose and has numerous potential applications, both as a building block chemical and in direct end uses. The unique molecular structure of glucaric acid, a carbohydrate diacid, provides for a range of technical applications that require varying levels of solubility, biodegradability, and safe dispersal in the environment. Conventionally, glucaric acid is made from glucose using nitric acid as the oxidizing agent. Other aldaric acids are created from aldoses in a similar manner. In recent years, other oxidation methods for preparing glucaric acid have been developed. Nitric acid, however, remains superior with respect to versatility, reaction efficiency, both in time and energy, and in raw material cost.

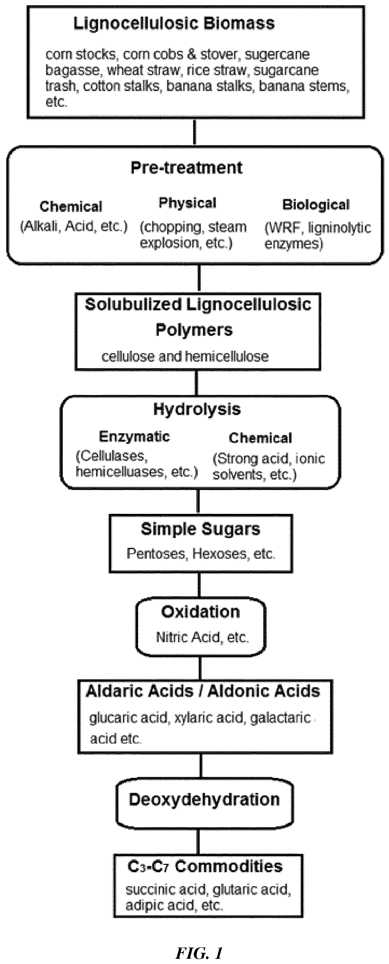

The high degree of oxygenation of sugar derivatives, like aldaric acids, creates a synthetic challenge. Deoxydehydration (DODH) reactions provide a potential solution. Traditionally, DODH reactions have been implemented for the conversion of vicinal diols to olefin products using high valent oxo-rhenium catalysts with various reducing agents (*e.g*., see **FIG. 1**).

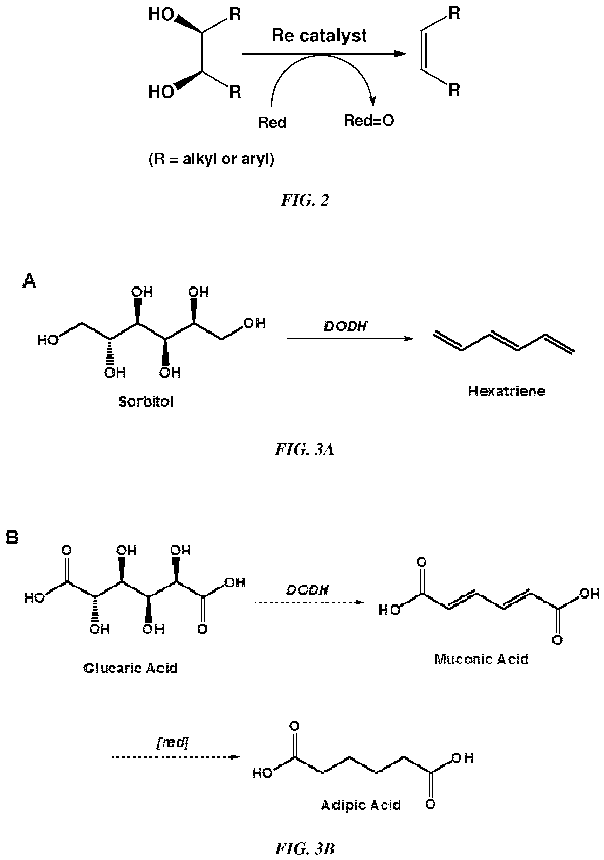

The application of DODH to industrially relevant processes, however, requires further modifications, namely the incorporation of H₂ gas as the reducing source. The main advantage of using H₂ gas is the generation of H₂O as the sole byproduct of the catalytic cycle. While DODH has been shown to be reduced by H₂, the results have been greatly limited by substrate scope, modest yields, and the over-reduction of the resulting olefin. In addition to the incorporation of H₂ gas, the ability to perform DODH in the presence of carboxylic acids and ester motifs is also advantageous. The DODH reaction with sorbitol, the reduced derivative of glucose, provides hexatriene, which to date, has not found much industrial utility (see **FIG. 2A**). The ability to perform DODH with oxidized derivatives of glucose (*i.e*., glucaric acid motifs) would generate muconic acid. A simple olefin reduction would then give adipic acid (see **FIG. 2B**), which has huge industrial relevance.

The disclosure provides for DODH methods capable of reducing a sugar derivative to a reduced DODH olefin adduct which can be further reduced to an unsaturated dicarboxylic acid product or an unsaturated di-ester product. In particular embodiments, the DODH methods disclosed herein provide for a reaction mixture comprising a sugar derivative or a sugar compound, a catalyst, a reducing agent, and a solvent system. Typically, the reaction is heated and maintained at an elevated temperature for a sufficient period of time to allow for product formation. However, depending upon the components of the reaction mixture, such as the sugar derivative used or the catalyst used, product formation may still result without the use of supplemental heating, by maintaining the reaction at or around ambient temperature or at a lower temperature. In further embodiments, the reaction is maintained at temperature from 20°C to 300°C, 50°C to 250°C, from 100°C to 180°C, or from 120°C to 160°C. In a particular embodiment, the reaction mixture is heated and maintained at a temperature around 150°C. Generally, the reaction is performed up to 72 hours, up to 48 hours, up to 24 hours, up to 12 hours, up to 6 hours, up to 3 hours, from 30 minutes to 3 hours, from 1 to 2 hours at a certain temperature (*e.g.*, around 150°C).

In some embodiments, it is preferred to first apply a lower temperature, i.e., about 20°C to about 130°C, then raise the temperature to complete the reaction. In particular, certain substrates cannot withstand an initial high temperature and decompose. For these substrates, a "thermal posttreatment" significantly increased the yield of the desired product.

In a particular embodiment, the DODH methods disclosed herein utilize a sugar derivative as a substrate. Examples of sugar derivatives, include, but are not limited to, aldaric acids, derivatives of aldaric acids (*e.g*., ester substituted aldaric acids), aldonic acids, derivatives of aldonic acids (*e.g*., ester substituted aldonic acids), sugar lactones (*e.g*., ribonolactone), and derivatives of sugar lactones.

In a certain embodiment, the DODH methods disclosed herein utilizes a catalyst (*e.g*., a transition metal-based catalyst). In a further embodiment the catalyst is an oxorhenium based catalyst. Examples of oxorhenium based catalysts, include, but are not limited to, HReO₄, KReO₄, NH₄ReO₄, ReO₂, ReIO₂(Ph₃P)₂, ReCl₃O(Ph₃P)₂, CH₃ReO₃ (MTO), and ReCl₃. In a further embodiment, the catalyst used in the methods of the disclosure is MTO. In an alternate embodiment, the catalyst used in methods disclosed herein is a vanadium-based catalyst. Examples of vanadium-based catalysts include, but are not limited to, NBu₄VO₃, NBu₄VO₂(CA)₂, HC(PZ)VO₂BF₄, TpaVO₂PF₆, NaVO₂(acac)₂, and Bu₄N(dipic)VO₂. In a particular embodiment, the vanadium-based catalyst is Bu₄N(dipic)VO₂ (dioxovanadium(v)dipicolinate). In yet another alternate embodiment, the catalyst used in methods disclosed herein is a molybdenum-based catalyst. Examples of molybdenum-based catalysts include, but are not limited to, MoO₃, Mo(CO)₆, Mo(CO)₄(bipy), MOO₂Cl₂(bipy), MoO₂Br₂(bipy), MoO₂(CH₃)₂(bipy),(NH₄)₆Mo₇O₂₄·4H₂O, H₃PMo₁₂O₄₀, and (NH₄)₆MO₇O₂₄·4H₂O. In a certain embodiment, the molybdenum-based catalyst is Mo(CO)₄(bipy) or (NH₄)₆Mo₇O₂₄·4H₂O. In a further embodiment, the catalyst can be loaded as low as 2.5% and still provide acceptable yields.

In one preferred embodiment, the catalyst comprises KReO₄ in combination with Pd/C. The KReO₄-Pd/c combination allows the use of a low H₂ pressure, which generate a saturated lactone.

The DODH methods disclosed herein can further utilize one or more additional reducing agents in addition to hydrogen gas. Examples of such reducing agents, include, but are not limited to, sodium sulfite, triphenylphospine, and secondary alcohols.

The DODH methods of the disclosure typically use a solvent system. Examples of solvents that can be used in the methods disclosed herein, include, but are not limited to, alcohols (*e.g*., methanol, ethanol, isopropanol, *n*-propanol, and *n*-butanol), carboxylic acids (*e.g*., formic acid, acetic acid, *p*-toulenesulfonic acid), water, nonpolar organic solvents (*e.g*., toluene, benzene, xylene, hexane, diethyl ether, dichloromethane, and 1,4-dioxane), polar organic solvents (*e.g*., tetrahydrofuran, ethyl acetate, acetone, dimethylformamide, acetonitrile, and dimethyl sulfoxide).

In further embodiments, it has been shown that the addition of an acid to the reaction mixture described herein may allow for the full conversion of a reactant (*e.g*., a sugar lactone derivative) to a desired product (*e.g*., diethyl adipate) without having to use differential H₂ pressures. Examples of acids that can be used include but are not limited to, phosphoric acid, hydrochloric acid, hydrofluoric acid, nitric acid, nitrous acid, acetic acid, sulfuric acid, citric acid, carbonic acid, oxalic acid, and formic acid. It is a particular benefit of this embodiment that the acidic additive allows opening of the saturated lactone obtained formed after the first DODH/hydrogenation cycle, and thus adipic acid or its ester is formed be formed after a second DODH/hydrogenation cycle.

In further embodiments, it has been shown that the addition of an activated charcoal to the reaction mixture described herein may allow for the full conversion of a reactant (e.g., a sugar lactone derivative) to a desired product (e.g., diethyl adipate) without having to use differential H₂ pressures and improved yields. An example of a charcoal that can be used is C270C purchased from Fischer.

In a certain embodiment, the DODH methods disclosed herein utilize an oxorhenium catalyst (KReO₄) and a hydrogen activating catalyst (Pd/C) in an alcoholic solvent (methanol) to convert a reactant (e.g., a sugar lactone derivative) to a desired product (e.g., dialkyl adipate) with hydrogen as the reducing agent at a temperature of 150 °C in yields >85%.

The following examples are intended to illustrate, but not limit, the disclosure. While they are typical of procedures that might be used, other procedures known to those skilled in the art may alternatively be used.

### EXAMPLES

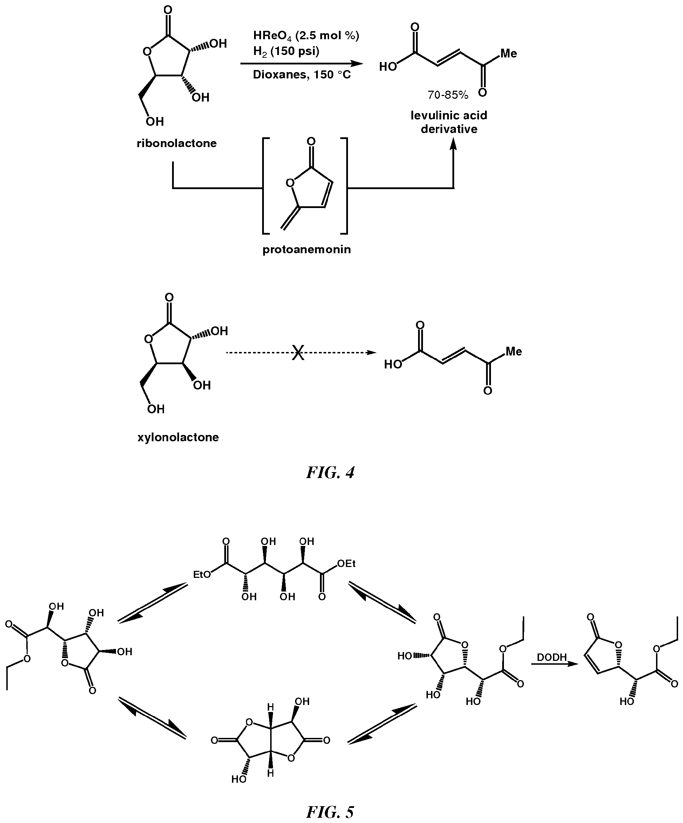

*Development of an effective DODH strategy to produce a DODH adduct lactone product from oxidized derivatives of glucose using H₂ as a reducing agent.* It was found that the unsaturated analog of levulinic acid could be isolated in moderate to good yield by performing a reaction with ribonolactone, a catalytic amount of HReO₄ in dioxanes at 150°C and under 150 psi of H₂ gas (see FIG. 4). It is theorized, but not relied upon, that this reaction occurs through a DODH reaction followed by an elimination reaction to generate protoanemonin. Under the reactions conditions, protoanemonin undergoes ring opening with residual water and subsequent olefin isomerization leads to the observed product. No reaction was observed when xylonolactone was exposed to the same reaction conditions. This was not surprising considering previous literature has shown that DODH reactions with cyclic diols require the syn stereochemistry found within ribonolactone, which is not present in xylonolactone.

*Modifying the previous DODH reaction conditions in view of the ribonolactone reduction findings.* In view of the foregoing results with ribonolactone, it was concluded that H₂-driven DODH is plausible with sugar-based derivatives. Unfortunately, the relative abundance of ribose is much lower than glucose.

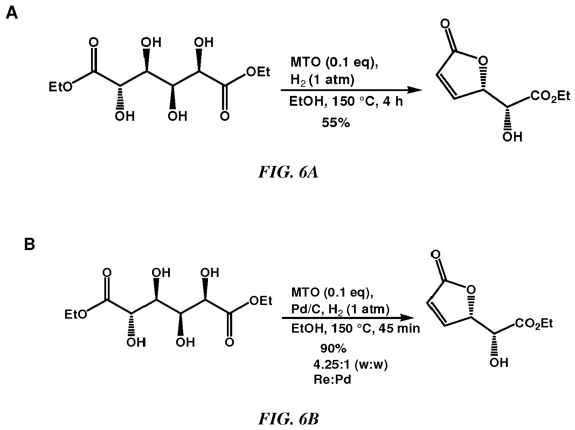

In a series of experiments, it was found that lowering the H₂ pressure of the reaction mixture facilitated increased yields of a DODH adduct resulting from glucaro-6,3-lactone (see **FIG. 5**). Additionally, it was found that the addition of 10% Pd/C facilitated not only a faster reaction, but an increased reaction yield as well (see **FIG. 6A** vs. **6B**).

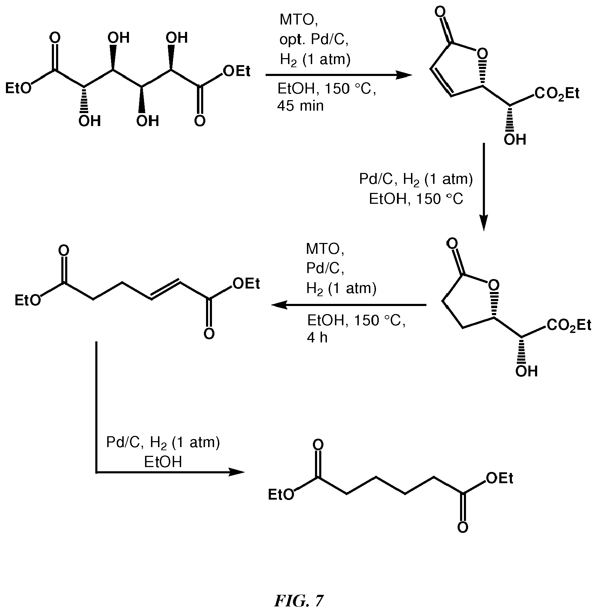

**Development of a one-pot strategy to form adipic acid from the DODH adduct lactone product.** After the successful production of the DODH lactone adduct, a process amenable to the formation of adipic acid from the DODH lactone adduct was devised. The unsaturated lactone generated from the first DODH event could be hydrogenated with fresh Pd/C under H₂ in quantitative yield. The saturated lactone was then subjected to nearly identical DODH conditions as previously utilized, which facilitated lactone ring opening and subsequent DODH with the resulting diol to provide an enoate in 95% yield. Lastly, olefin hydrogenation generated diethyl adipate in 97% yield (*e.g*., see **FIG. 7**).

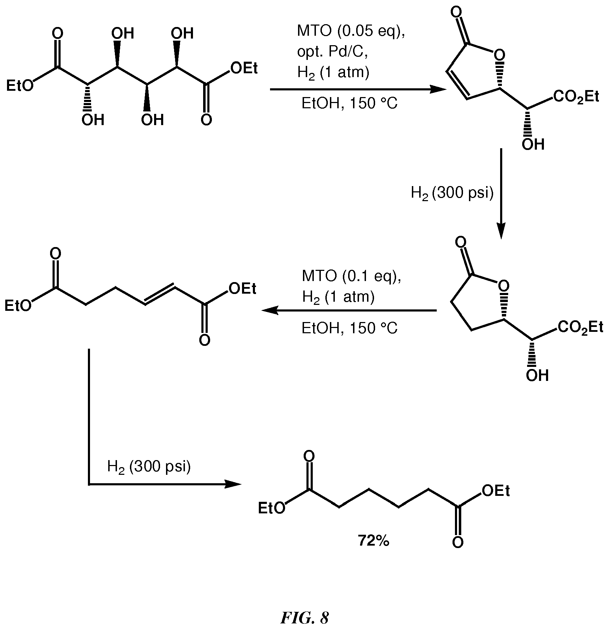

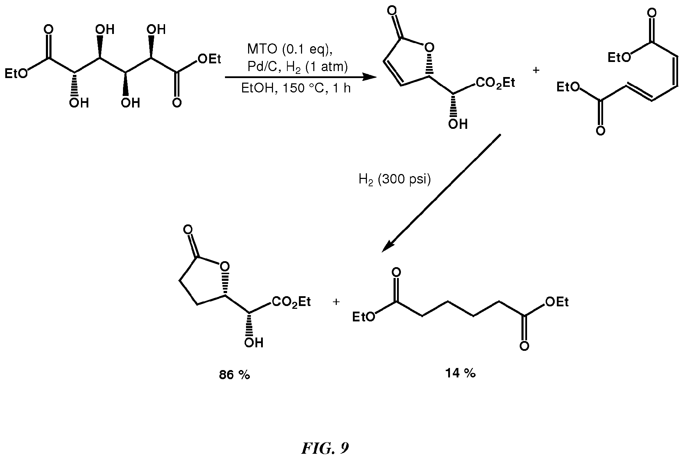

Based upon the synthetic route above, the similarity of the reaction conditions facilitated a one-pot sequence for the chemical transformation of diethyl glucarate to diethyl adipate. Palladium and rhenium catalysts added at various times during the reaction course led to complicated reaction mixtures. It was found, however, that modulation of the H₂ pressure combined with one additional loading of rhenium (*i.e.* MTO), provided diethyl adipate in 72% overall yield (NMR yield) (see **FIG. 8**). The overall yield was likely 10-15% higher due to the repetitive removal of reaction aliquots for reaction monitoring. The one-pot sequence was made possible with the finding that an increase in H₂ pressure following the completion of DODH reactivity enabled olefin hydrogenation to occur (see **FIG. 9**).

**DODH reactions:** A solution of the DODH substrate, MTO, and 10% Pd/C (4.25:1 Re:Pd, *i.e.* equal wt % of MTO to Pd/C) in EtOH (typically about 0.08M w/respect to a sugar derivative) was introduced into a Parr reactor or pressure sealed glass tube and purged with H₂ (1 atm), closed, and heated to 150°C. The reaction times vary depending on catalyst loadings, e.g., for 10 mol% MTO leads to about 1 hour, or 2 hours. The reaction was monitored by NMR analysis following the concentration of small reaction aliquots. When the reaction was observed to be complete, the reaction mixture was filtered through celite. The celite was rinsed with MeOH and the resulting liquid was concentrated.

**All-in-one pot synthesis:** A solution of diethyl glucarate, MTO, Pd/C in EtOH in a Parr reactor was setup as stated above. When the reaction was observed to be complete, the H₂ pressure was increased to 300 psi and reheated to 150°C. The reaction mixture was again monitored by NMR. Once olefin hydrogenation was complete, an additional loading of MTO was added to the reaction mixture. Typically, twice the loading of MTO compared to the initial loading was found to be sufficient DODH reactivity. The reaction was subjected to 1 atm H₂ and heated to 150°C. Again, the reaction mixture was monitored by NMR. Once complete, the reaction was pressurized to 300 psi H₂ and reheated to 150°C. When completed by NMR analysis, the reaction mixture was filtered through celite, rinsed with MeOH, and concentrated.

**NMR yield analysis:** the crude mixtures were evaluated with a known amount of mesitylene. Purification by column chromatography was typically performed with 0-3% MeOH:DCM, depending the extent of oxygenation of the product.

To further demonstrate the present DODH method, a series of experiments was performed to show that under the disclosed reactions conditions, only vicinal diols that are in α,β-position to an electron-withdrawing group, preferably a carbonyl group, and still more preferably a carboxylic acid, ester, or lactone moiety, undergo DODH.

The following three starting materials (SM) individually were subjected to the following reaction condition.

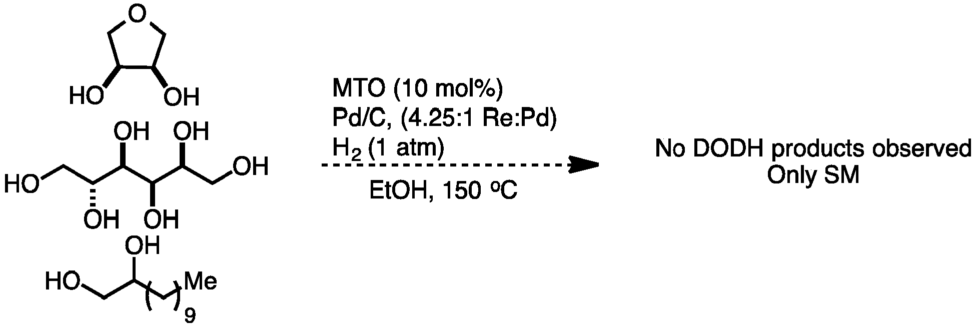

It was found that when no carboxylate group is present, then no conversion to DODH products occurred.

In another experiment, the mixture below was subjected to the indicated reaction conditions.

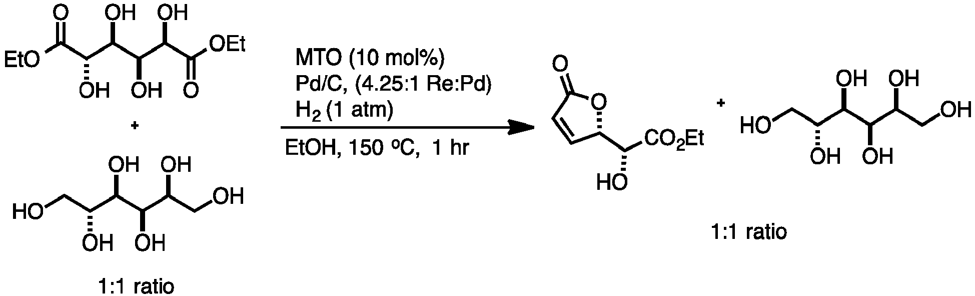

It was found that the disclosed catalyst system and reaction conditions selectively convert only vicinal diols that are α,β to a carboxylate group. The sorbitol was not converted.

The following substrates were subjected to the following DODH reaction. A solution of the DODH substrate, MTO, and 10% Pd/C (4.25:1 Re:Pd, *i.e.* equal wt % of MTO to Pd/C) in EtOH (typically about 0.08M with respect to a sugar derivative) was introduced into a Parr reactor or pressure sealed glass tube and purged with H₂ (1 atm), closed, and heated to 150°C. The reaction times vary depending on catalyst loadings, e.g. for 10 mol % MTO leads to about 1 hour, or 2 hours. The reaction was monitored by NMR analysis following the concentration of small reaction aliquots. When the reaction was observed to be complete, the reaction mixture was filtered through celite. The celite was rinsed with MeOH and the resulting liquid was concentrated.

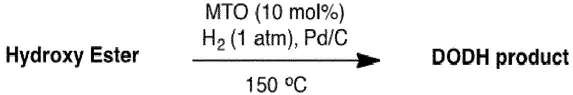

| Substrate | Product | Substrate | Product |
|---|---|---|---|
| 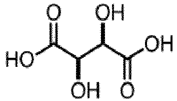 | 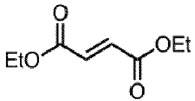 | 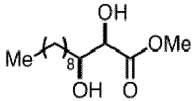 | 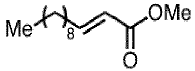 |
| **Tartaric Acid** | **74%** brsm | | **75%** |
| 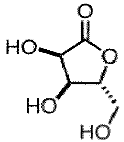 | 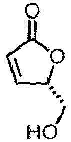 | 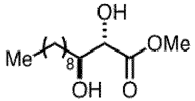 | 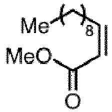 |
| **Ribonolactone** | **79%** | dr 4:1 | **Z:E 2:1** |
| | | 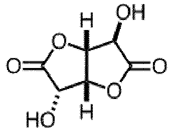 | 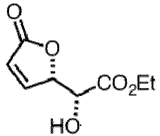 |
| | | **Glucaro-1,4:6,3-dilactone** | **66%** |

The above further illustrates the substrate selectivity of the disclosed DODH method.

In another experiment, a transformation to adipic acid in one pot was acheived, without adding additional catalyst, by regenerating the catalyst *in situ* by reoxidation with oxygen. As shown in the following reaction scheme, iteratively repeating the atmosphere exchange cycles, the yield of adipic acid was improved. The hydrogenation step is conducted at elevated H₂ pressure; whereas the DODH chemistry is conducted at low H₂ pressure.

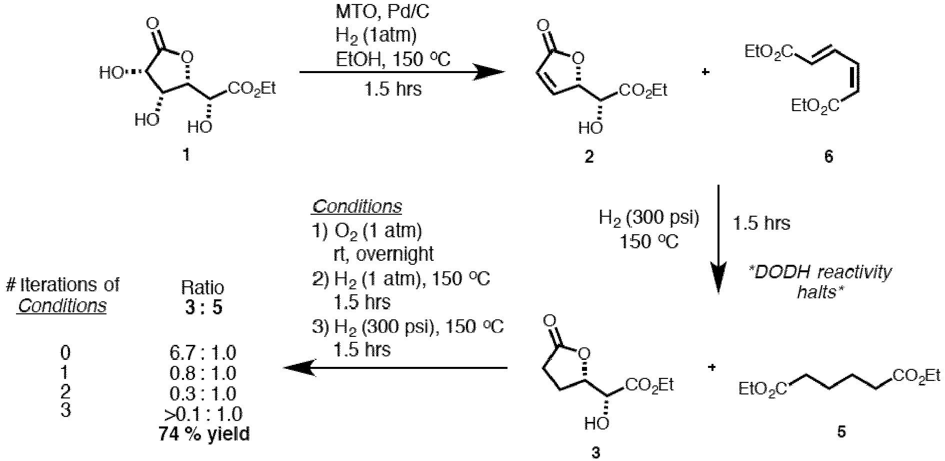

### Experimental:

The headspace above a solution of **1** (132 mg), MTO (15 mg), and 10% Pd/C (15 mg) in EtOH (7.5 mL) in a Parr reactor was purged with H₂ and maintained under 1 atm of H₂. The reaction was heated to 150°C. After 1.5 hours, the reaction was cooled to room temperature and the reactor was pressurized to 300 psi with H₂. The reaction was re-heated to 150°C for 1.5 hours. The reaction was cooled to room temperature. The headspace was purged with N₂ to facilitate removal of residual H₂. The headspace was then purged with 1 atm O₂, capped, and stirred at room temperature overnight. The reaction was purged with N₂ to remove residual O₂. The process described above (*i.e*., heating at 1 atm H₂, heating at 300 psi H₂, followed by O₂ treatment) was repeated 3 times.

The reaction mixture was collected, centrifuged, and the supernatant was removed by pipet. The remaining solids were washed and re-centrifuged with EtOH (2 x 10 mL). The combined supernatant was collected and concentrated to provide **5** in 74% yield (NMR analysis with mesitylene as a standard).

The following examples utilizes KReO₄ as a DODH catalyst. It was found that potassium perrhenate demonstrated a significant improvement compared to MTO in the DODH reaction.

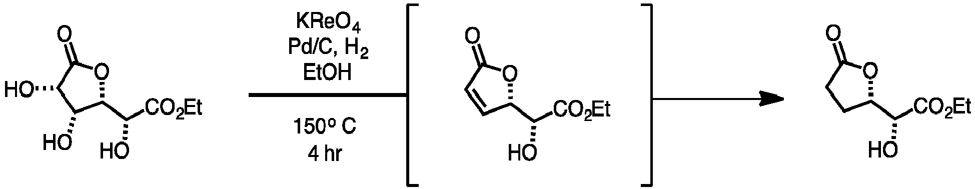

After 4 hours, full conversion of the starting material was achieved. In contrast to the MTO-Pd/C catalyst system, the hydrogenation activity was retained at low H₂ pressure leading to formation of the saturated lactone. Both the DODH reaction and the hydrogenation reaction occur with a H₂ pressure of 75-100 psi. Essentially only the desired product was observed in NMR analysis.

It therefore is possible to directly transform a glucaric acid derivative to adipic acid under one set of conditions, if the saturated lactone can be ring-opened and the second DODH performed.

### Experimental:

A Parr reactor charged with polyol (7.5 mmol), KReO₄ (22 mg), 10% Pd/C (60 mg), 85% H₃PO₄ (26 mg), and EtOH (7.5 mL) was pressurized to 75 psi with H₂. The reaction was placed in a preheated oil bath set to 150°C for a 4 hours. The reaction mixture was cooled to room temperature, filtered, rinsed with ethanol, and concentrated.

Mesitylene was added to the crude residue as a standard for the determination of yields by NMR.

The effect of including an acidic additive also was investigated. It was hypothesized that an acidic additive would allow opening of the saturated lactone obtained above, and thus adipic acid would be formed after a second DODH/hydrogenation cycle.

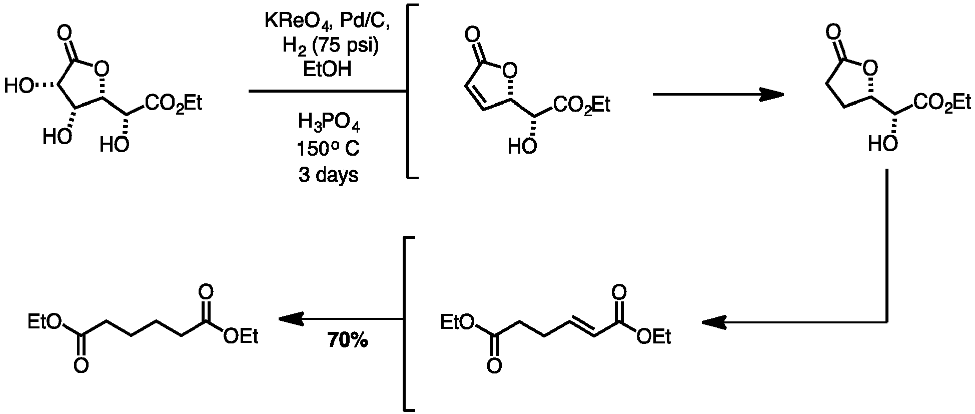

The effect of phosphoric acid addition is illustrated in the above reaction system. Under these acidic conditions, the hypothesis was proved correct. The saturated lactone reacts further, and diethyl adipate was formed in 70% overall yield from the starting material.

### Experimental:

A Parr reactor charged with polyol (7.5 mmol), KReO₄ (22 mg), 10% Pd/C (60 mg), 85% H₃PO₄ (26 mg), and EtOH (7.5 mL) was pressurized to 75 psi with H₂. The reaction was placed in a preheated oil bath set to 150°C until the reaction was complete (typically from a few hours up to three days). The reaction mixture was cooled to room temperature, filtered, rinsed with EtOH, and concentrated.

Mesitylene was added to the crude residue as a standard for the determination of yields by NMR.

In another example, it was found that activated charcoal as an additive significantly promoted the DODH/hydrogenation reaction. The exemplary charcoal is C270C purchased from Fischer. The experiments were performed on a series of substrates shown in the following table.

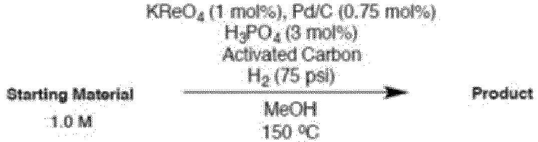

| Substrate | Product | Substrate | Product |
|---|---|---|---|
| 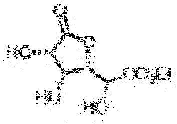 | 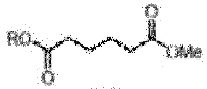 | 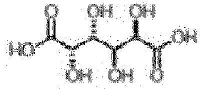 | 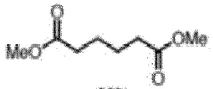 |
| | 91% | Mucic Acid | 86% |
| 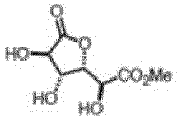 | 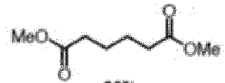 | 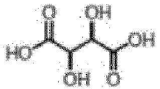 | 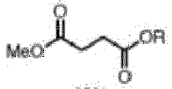 |
| | 86% | Tataric Acid | 88% |
| 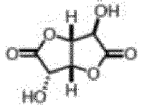 | 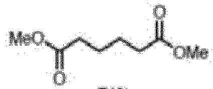 | 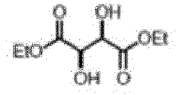 | 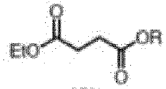 |
| lucaro-1,4:6,3-dilactone | 71% | | 95% |
| | 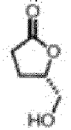 | 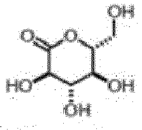 | 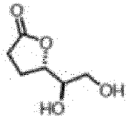 |
| 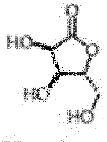 | 75% | 1,6-Gluconolactone | 60% |
| Ribonolactone | 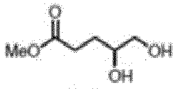 | | |
| | 22% | | |

The table above demonstrates the significant yield improvements achieved by the addition of charcoal. The yield of adipate was improved to 91% from 70% by addition of activated charcoal, while the same ease of use (no atmosphere exchange, all reactions to the adipate carried out under the same set of conditions) was maintained. The catalyst system is also competent in the transformation of other diols in α,β-position to carboxylate moieties as demonstrated in the table above. The final example in the table, i.e., 1,5-gluconolactone, demonstrates this selectivity within a single molecule: only the α,β-diol group is transformed. The other hydroxy functionalities are not affected.

### Experimental:

A Parr reactor charged with polyol (7.5 mmol), KReO₄ (22 mg), 10% Pd/C (60 mg), 85% H₃PO₄ (26 mg), granular activated carbon (450 mg, C270C, purchased from Fisher), and MeOH (7.5 mL) was pressurized to 75 psi with H₂. The reaction was placed in a preheated oil bath set to 150°C for a given amount of time until the reaction was complete (typically from a few hours up to three days). The reaction mixture was cooled to room temperature, filtered, rinsed with MeOH, and concentrated.

Mesitylene was added to the crude residue as a standard for the determination of yields by NMR.

The effect of temperature and substrate on activity and catalyst loading also was investigated. Using the conditions disclosed above, the influence of an increase in temperature was examined. When using 6,3-glucarolactone monoethyl ester as the starting material, the temperature can be increased with essentially no loss of yield (90% vs. 91%) and with a decrease in reaction to 7 hours from 18 hours as shown below. The reaction at lower temperature was carried out with KReO₄ as the rhenium source, whereas the higher temperature reaction used (NBu₄)ReO4.

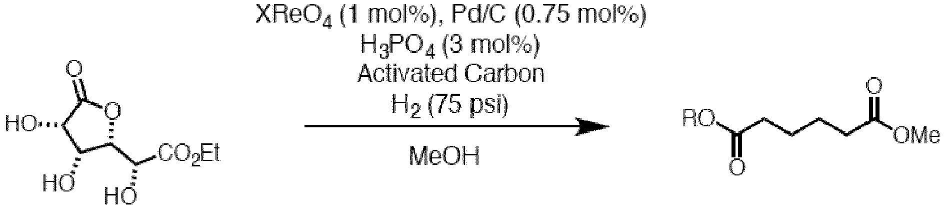

| Temperature ( ºC) | Rxn Time | % Yield |
|---|---|---|
| 150 | 18 hr | 91 |
| 170-175 | 7 hr | 90 |

### Effect of Temperature

When using glucarodilactone as the starting material, direct application of 170°C led to significant product decomposition. This was overcome by first applying a lower temperature (120°C) to the reaction mixture and subsequently raising the temperature to 170°C. Using this "thermal pre-treatment" approach, dimethyl adipate was obtained in 90% yield compared to 70% when the reaction was carried out at 150°C. Using the thermal pre-treatment reaction conditions, the catalyst loading can be reduced from 1 mol% KReO₄ to 0.25 mol% with only a moderate decrease in yield (75% vs. 90%).

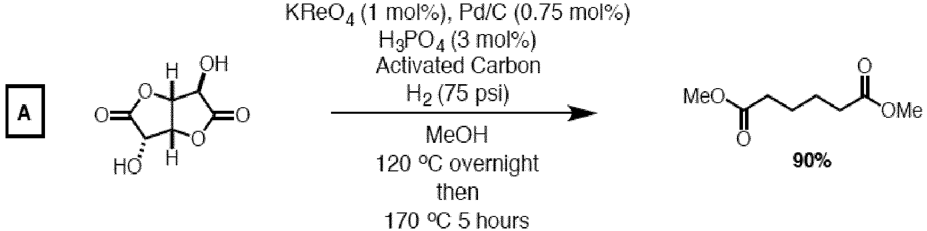

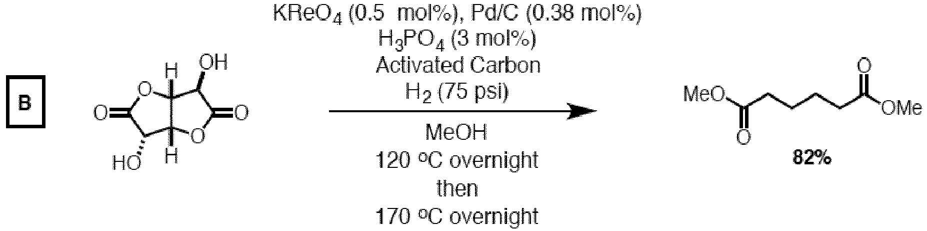

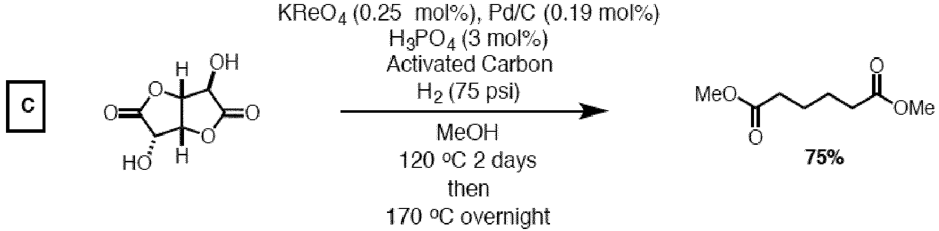

### Lower Catalyst Loadings

### Experimental:

Representative procedure: A Parr reactor charged with polyol (7.5 mmol), KReO₄ (22 mg), 10% Pd/C (60 mg), 85% H₃PO₄ (26 mg), granular activated carbon (450 mg, C270C, purchased from Fisher), and MeOH (7.5 mL) was pressurized to 75 psi with H₂. The reaction was placed in a preheated oil bath set to the appropriate temperature for a given amount of time. The reaction mixture was cooled to room temperature, filtered, rinsed with MeOH, and concentrated.

Mesitylene was added to the crude residue as a standard for the determination of yields by NMR.

In another example, a Parr reactor charged with polyol (7.5 mmol), KRe04 (22 mg), 10% Pd/C (60 mg), 85% H3PO4 (26 mg), and EtOH(7.5 mL) was pressurized to 75 psi with H2. The reaction was placed in a preheated oil bath set to 150°C until the reaction was complete (typically from a few hours up to three days). The reaction mixture was cooled to room temperature, filtered, rinsed with EtOH, and concentrated.

Mesitylene was added to the crude residue as a standard for the determination of yields by NMR. It was found that diethyl adipate was formed in 70% overall yield from the starting material.

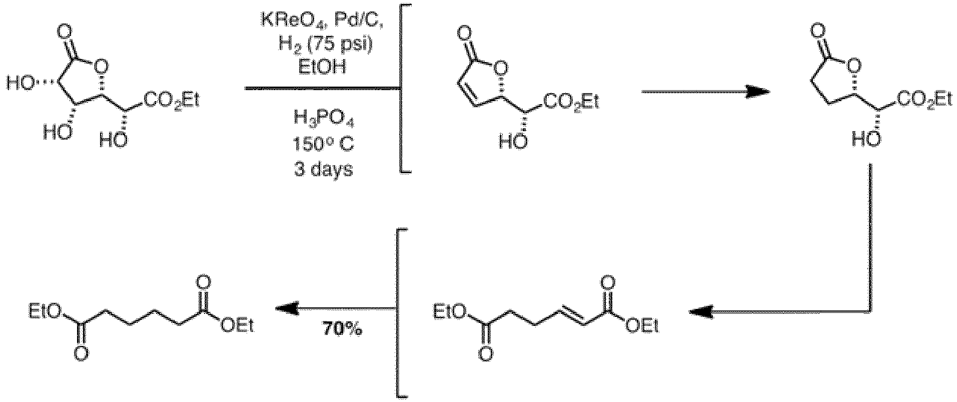

The effect of solvent and substrate also was investigated. It was found that the choice of solvent has a strong effect on the yield of the reaction when glucarodilactone is used as the substrate.

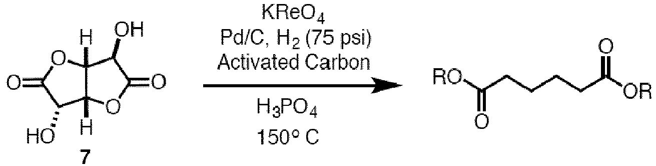

| Solvent | Concentration | Mol % of Re and Pd | Mol % of H₃PO₄ | Yield (%) |
|---|---|---|---|---|
| EtOH | 0.75 M | 1.5 %, 1.2 % | 1.5 % | 66 % |
| EtOH* | 1.0 M | 1.0 %, 0.75 % | 3.0 % | 67 % |
| MeOH* | 1.0 M | 1.0 %, 0.75 % | 3.0 % | **78 %** |

| | | | | |
|---|---|---|---|---|
| * Required thermal treatment at 120 °C for 1.25 hours prior to addtion of catalyst system | | | | |

Using methanol as the solvent improves the yield by about 10% (78% vs 67%). It is theorized, but not relied upon, that, in methanol, glucarodilactone is rapidly converted into either the monolactone monoethyl ester species or the dimethyl glucarate. These compounds are theorized to be less thermally sensitive than the dilactone, thus reducing decomposition over time. To test this theory, the dilactone was reacted for 1.25 h at 120°C in ethanol and methanol, respectively, and the product distribution analyzed.

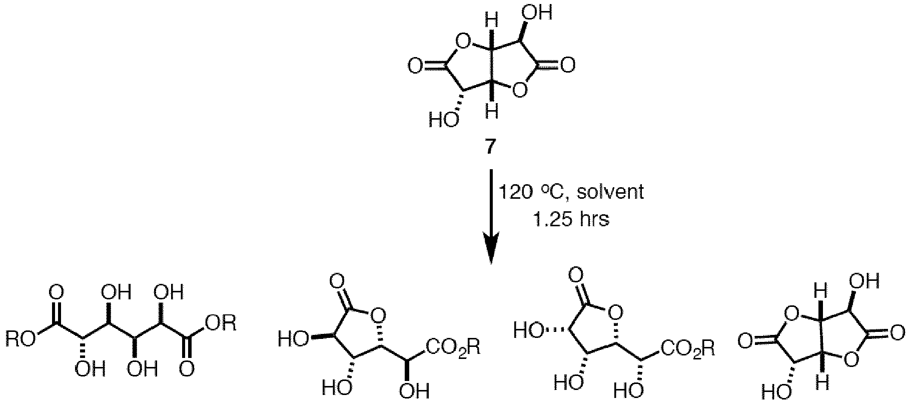

| **Solvent** | **Ratio following thermal treatment** | | | |
|---|---|---|---|---|
| EtOH | 2.5 | 2.9 | 2.9 | 1.0 |
| MeOH | 5.3 | 4.0 | 4.0 | 1.0 |

When methanol was used as the solvent, a stronger shift of the product distribution towards the diester and the monoesters was detected compared to the use of ethanol. Thus, a low temperature thermal pre-treatment is advantageous when using glucarodilactone as the starting material with an alcohol as the solvent. Methanol is a preferred alcoholic solvent.

### Experimental:

DODH reaction: Representative procedure: A Parr reactor charged with polyol (7.5 mmol), KReO₄ (22 mg), 10% Pd/C (60 mg), 85% H₃PO₄ (26 mg), granular activated carbon (450 mg, C270C, purchased from Fisher), and MeOH (7.5 mL) was pressurized to 75 psi with H₂. The reaction was placed in a preheated oil bath set to the appropriate temperature for a given amount of time. The reaction mixture was cooled to room temperature, filtered, rinsed with MeOH, and concentrated.

Mesitylene was added to the crude residue as a standard for the determination of yields by NMR.

As demonstrated above, an important feature of the present invention is the discovery that the addition of a second component to the catalyst system, e.g., Pd/C, surprisingly improved the DODH capabilities of the catalyst system.

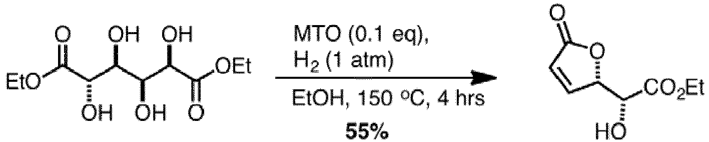

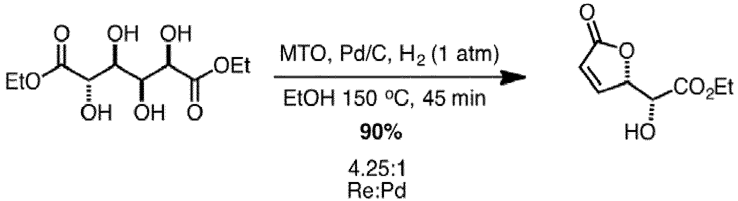

The above reactions demonstrate that the addition of the Pd/C increases the reaction speed (4 hours to 0.75 hours) and the yield to 90% from 55%.

The same results were obtained when using the 6,3-glucarolactone monomethyl ester (lactone 2 below) as the substrate. It was found that omitting either the hydrogen or the hydrogenation catalyst results in significantly lower yields.

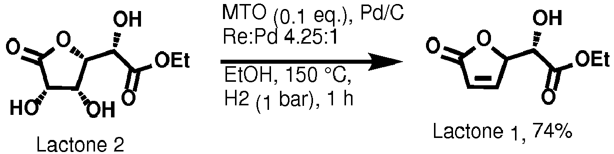

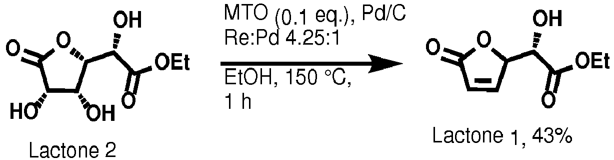

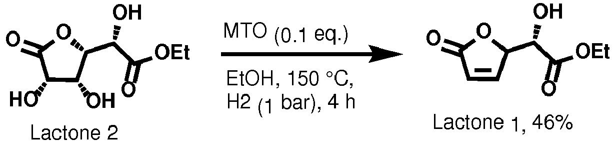

Another important feature of the present invention is the discovery of a four step route to diethyl adipate. In particular, diethyl adipate can be obtained from lactone 1 in a four step reaction sequence coupling DODH reaction steps and hydrogenation steps.

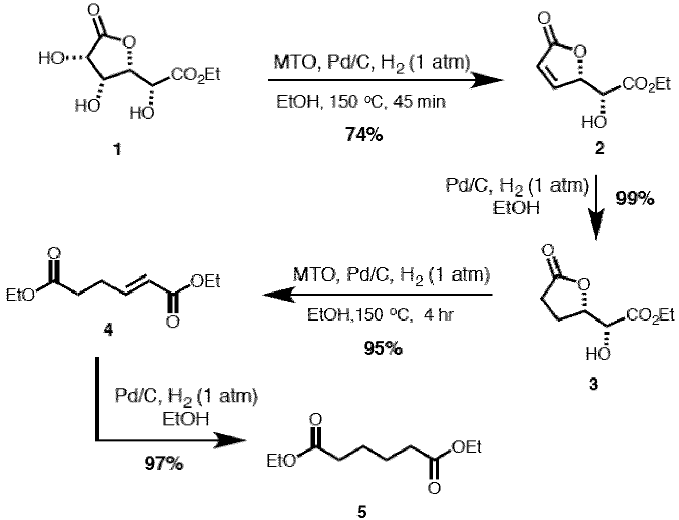

A 350 mL glass schlenk tube was charged with **1** (450 mg), MTO (21 mg), 10% Pd/C (21 mg), and EtOH (21 mL). The tube was purged with H₂ and capped with 1 atm of H₂. The reaction was placed in a preheated oil bath at 150 °C. After 2.5 hours, the reaction was cooled to room temperature. The mixture was filtered through celite, rinsed with MeOH, and concentrated. The crude residue was purified by column chromatography (2.5% MeOH:DCM) to provide compound **2** (282 mg, 74%) as a white solid.

A solution of compound **2** (100 mg) and 10% Pd/C (15 mg) in EtOH (5 mL) was stirred under 1 atm of H₂ (balloon) for 2 hours at room temperature. The reaction was filtered through celite, rinsed with EtOH, and concentrated to provide **3** (100 mg, 99%) as a white solid.

A 100 mL glass schlenk tube was charged with **3** (80 mg), MTO (10 mg), 10% Pd/C (4 mg), and EtOH (5 mL). The tube was purged with H₂ and capped with 1 atm of H₂. The reaction was placed in a preheated oil bath at 150 °C. After 4 hours, the reaction was cooled to room temperature, filtered through celite, rinsed with DCM, and concentrated. The crude residue was purified by column chromatography (DCM) to provide **4** (81 mg, 95%) as a colorless oil.

A solution of **4** (68 mg) and 10% Pd/C (5 mg) in EtOH (2 mL) stirred under 1 atm of H₂ (balloon) overnight. The reaction was filtered through celite, rinsed with DCM, and concentrated to provide **5** (63 mg, 97%) as a colorless oil.

The invention will now further be described by further embodiments:

### EMBODIMENTS

1. A method for the deoxydehydration (DODH) of a sugar derivative, comprising:
   (a) incubating a reaction mixture for a sufficient period of time to allow for formation of one or more deoxydehydrated products, wherein the reaction mixture comprises:
      a reactant selected from the group consisting of an aldaric acid, an aldaric acid derivative, an aldonic acid, aldonic acid derivative, a sugar lactone, and a sugar lactone derivative;
      a catalyst selected from the group consisting of a vanadium-based catalyst, a molybdenum-based catalyst, a rhenium-based catalyst, and any combination thereof;
      a reducing agent comprising hydrogen gas;
      a solvent system; and
      optionally an acid.
2. The method of embodiment 1, wherein the reaction is carried out by incubating the reaction mixture at a temperature greater than 20°C.
3. The method of embodiment 2, wherein the reaction mixture is incubated at a temperature between 120°C to 300°C.
4. The method of any one of the preceding embodiments, wherein the reaction is carried out for up to 72 hours.
5. The method of any one of the preceding embodiments, wherein the reaction mixture is incubated at about 150°C for up to 4 hours.
6. The method of any one of the preceding embodiments, wherein the catalyst can be regenerated and reused in step (a) by exposing the catalyst to an oxidizing agent comprising oxygen gas.
7. The method of any one of the preceding embodiments, wherein after performing step (a), the method further comprises:
   (b') adding to the reaction mixture a catalyst selected from the group consisting of a vanadium-based catalyst, a palladium-based catalyst, a platinum-based catalyst, a nickel-based catalyst, a molybdenum-based catalyst, a lithium-based catalyst, an aluminum based-catalyst, an iron-based catalyst, an iridium-based catalyst, a rhodium-based catalyst, a rhenium-based catalyst, and any combination thereof; and subsequently or simultaneously increasing the pressure of the hydrogen gas up to 300 psi and heating the reaction mixture at a temperature between 120°C to 160°C.
8. The method of embodiment 7, wherein the method is repeated one or more times.
9. The method of any one of the preceding embodiments, wherein the method further comprises separating the product from any remaining reactant and reaction intermediates.
10. The method of any one of the preceding embodiments, wherein the method is performed using a one pot synthesis strategy.
11. The method of any one of the preceding embodiments, wherein the one or more reduced product(s) comprises at least one reduced product that having a structure selected from the group consisting of formula I, formula II, formula III, and formula IV: 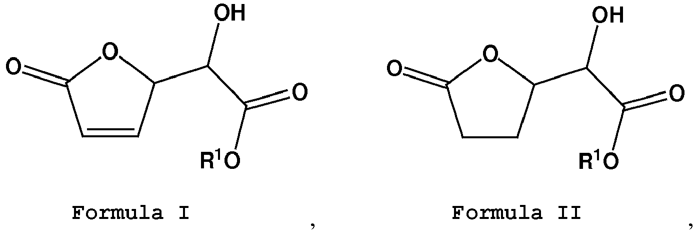 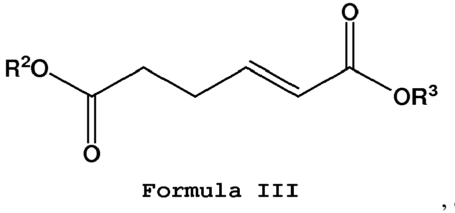 and 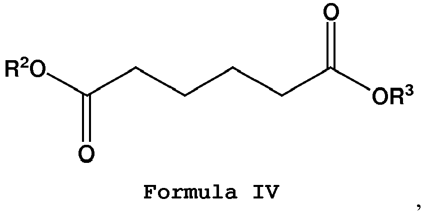 wherein,
   R¹, R², and R³ are each independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl.
12. The method of embodiment 11, wherein the reduced product of formula IV is produced from the reduced products of formula I, or from formula II that is produced from the compound of formula I or from formula III that is produced from formula II that is produced from formula I.
13. The method of embodiment 1, wherein after performing step (a), the method further comprises:
   (b) adding to the reaction mixture one or more catalyst suitable for hydrogenation of an alkene and/or increasing a pressure of hydrogen gas up to 300 psi; followed by
   (c) repeating step (a); followed by
   (d) repeating step (b); and
   (e) optionally repeating steps (a) and (b) until the majority of the sugar derivatives has been converted to a reduced product having the structure of formula IV: 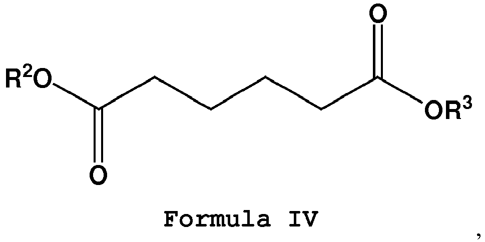 wherein,
      R¹, R², and R³ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl.
14. The method of embodiment 13, wherein steps (a), (b), (c), (d) and/or (e) are carried out at a temperature from 20°C to 300°C.
15. The method of any one of the preceding embodiments, wherein the reactant is an aldaric acid and the deoxydehydrated product is an unsaturated di-carboxylic acid compound.
16. The method of embodiment 15, wherein the reactant is glucaric acid and the one or more deoxydehydrated products is adipic acid.
17. The method of any one of the preceding embodiments, wherein the rhenium-based catalyst is selected from the group consisting of HReO₄, NH₄ReO₄, ReO₂, ReIO₂(Ph₃P)₂, ReCl₃O(Ph₃P)₂, CH₃ReO₃ (MTO), and ReCl₃.
18. The method of embodiment 17, wherein the rhenium-based catalyst is MTO or HReO₄.
19. The method of any one of the preceding embodiments, wherein the vanadium-based catalyst is selected from the group consisting of NBu₄VO₃, NBu₄VO₂(CA)₂, HC(PZ)VO₂BF₄, TpaVO₂PF₆, NaVO₂(acac)₂, and Bu₄N(dipic)VO₂.
20. The method of any one of the preceding embodiments, wherein the molybdenum-based catalyst is selected from the group consisting of MoO₃, Mo(CO)₆, Mo(CO)₄(bipy), MOO₂Cl₂(bipy), MoO₂Br₂(bipy), MoO₂(CH₃)₂(bipy),(NH₄)₆Mo₇O₂₄·4H₂O, and H₃PMo₁₂O₄₀.
21. The method of any one of the preceding embodiments, wherein the reaction mixture further comprises palladium on carbon (Pd/C), sodium sulfite, triphenylphospine, and/or secondary alcohols.
22. The method of embodiment 21, wherein the reaction mixture further comprises Pd/C.
23. The method of any one of the preceding embodiments, wherein the solvent system comprises ones or more polar solvent selected from the group consisting of water, methanol, ethanol, n-propanol, n-butanol, isopropanol, acetic acid, and formic acid.
24. The method of embodiment 23, wherein the solvent system comprises ethanol.
25. A method to produce (C₄-C₇)-linear saturated carboxylic acids from polysaccharides and/or disaccharides, comprising:
   (A) treating polysaccharides and/or disaccharides with enzymes to hydrolyze the polysaccharides and/or disaccharides into simple sugars;
   (B) oxidizing the simple sugars to form aldonic acids or aldaric acids; and
   (C) deoxydehydrating the aldonic acids or aldaric acids with the method of any one of the preceding embodiments to produce (C₄-C₇)-linear saturated carboxylic acids; or optionally
   (B') derivatize the aldonic acid or aldaric acid of step (B), and
   (C') deoxydehydrating the aldonic acid derivatives or aldaric acid derivatives with the method of any one of the preceding embodiments to produce (C₄-C₇) linear saturated carboxylic acids and/or (C₄-C₇)-linear saturated carboxylic acid derivatives, which may be hydrolyzed to the (C₄-C₇)-linear saturated carboxylic acids.
26. A method to produce (C₄-C₇)-linear saturated carboxylic acids from a lignocellulosic biomass, comprising:
   (A) pretreating the lignocellulosic biomass with one or more physical process, one or more chemical process, and/or one or more biological agent, or any combination thereof to generate solubilized lignocellulosic polymers;
   (B) hydrolyzing the lignocellulosic polymers using enzymes and/or chemical treatment to obtain simple sugars;
   (C) oxidizing the simple sugars to form aldaric acids or aldonic acids; and
   (D) deoxydehydrating the aldaric acids or aldonic acids with the method of any one of embodiments 1 to 24 to produce (C₄-C₇)-linear saturated carboxylic acids; or optionally
   (C') derivatize the aldonic acid or aldaric acid of step (C); and
   (D') deoxydehydrating the aldonic acid derivatives or aldaric acid derivatives with the method of any one of the preceding embodiments to produce (C₄-C₇)-linear saturated carboxylic acids and/or (C₄-C₇)-linear saturated carboxylic acids derivatives, which may be hydrolyzed to the (C₄-C₇)-linear saturated carboxylic acids.
27. The method of embodiment 26, wherein for (A), the lignocellulosic biomass is pretreated with one or more physical process and/or with acid; for (B), the lignocellulosic polymers are hydrolyzed by enzymatic action; and/or for (C), the simple sugars are oxidized by treating with nitric acid.
28. The method of embodiment 26 or embodiment 27, wherein the aldaric acids comprise glucaric acid, wherein the (C₄-C₇)-linear saturated carboxylic acid derivative comprises adipic acid esters, and wherein the (C₄-C₇)-linear saturated carboxylic acid comprises adipic acid.
29. The method of embodiment 1, wherein the reaction mixture comprises an aldaric acid derivative reactant having the structure of: 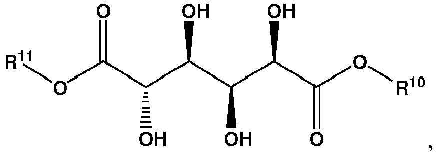 wherein,
   R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl, wherein at least one of R¹⁰ or R¹¹ is not H.
30. The method of embodiment 29, wherein the reaction mixture is incubated at a temperature greater than 20°C.
31. The method of embodiment 30, wherein the reaction mixture is incubated at a temperature between 120°C to 300°C.
32. The method of any one of embodiments 29 to 31, wherein the reaction mixture is incubated for up to 72 hours.
33. The method of any one of embodiments 29 to 32, wherein the reaction mixture is incubated at about 150°C for up to 4 hours.
34. The method of any one of embodiments 29 to 33, wherein the catalyst is a rhenium-based catalyst.
35. The method of embodiment 34, where the rhenium based catalyst is MTO.
36. The method of any one of embodiments 29 to 35, wherein the solvent system comprises an alcohol.
37. The method of embodiment 36, wherein the solvent system comprises ethanol.
38. The method of any one of embodiments 29 to 37, wherein the reaction mixture further comprises palladium on carbon (Pd/C).
39. The method of any one of embodiments 29 to 38, wherein the deoxydehydrated product of the deoxydehydration reaction comprises a lactone derivative having a structure of: 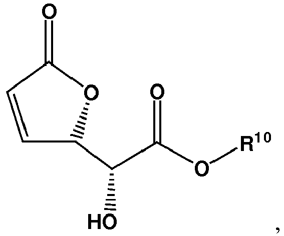 wherein,
   R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
40. The method of embodiment 39, wherein the method further comprises reacting the lactone derivative with one or more reducing agents comprising hydrogen gas.
41. The method of embodiment 40, wherein the hydrogen gas is used at a pressure up to 300 psi.
42. The method of embodiment 40, wherein the lactone derivative is reduced at a temperature greater than 20°C in a solvent system comprising a catalyst suitable for the hydrogenation of an alkene.
43. The method of embodiment 42, wherein the solvent system comprises an alcohol.
44. The method of embodiment 43, wherein the solvent system comprises ethanol.
45. The method of any one of embodiments 42 to 44, wherein the catalyst comprises Pd/C.
46. The method of any one of embodiments 40 to 45, wherein the reduction reaction produces a reduced lactone product having a structure of: 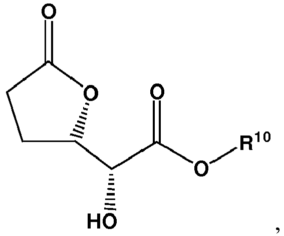 wherein,
   R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
47. The method embodiment 46, wherein the method further comprises deoxydehydrating the reduced product comprising,
   incubating a reaction mixture comprising the reduced product for a sufficient period of time to allow for formation of a deoxydehydrated product, wherein the reaction mixture comprises:
   a catalyst selected from the group consisting of a vanadium-based catalyst, a molybdenum-based catalyst, a rhenium-based catalyst, and any combination thereof;
   a reducing agent comprising hydrogen gas;
   a solvent system; and
   optionally an acid.
48. The method of embodiment 47, wherein the reaction is carried out by incubating the reaction mixture at a temperature greater than 20°C.
49. The method of embodiment 48, wherein the reaction mixture is incubated at a temperature between 120°C to 300°C.
50. The method of any one of embodiments 47 to 49, wherein the reaction is carried out for up to 72 hours.
51. The method of any one of embodiments 47 to 50, wherein the reaction mixture is incubated at about 150°C for up to 4 hours.
52. The method of any one of embodiments 47 to 51, wherein the rhenium-based catalyst is selected from the group consisting of HReO₄, NH₄ReO₄, ReO₂, ReIO₂(Ph₃P)₂, ReCl₃O(Ph₃P)₂, CH₃ReO₃ (MTO), and ReCl₃.
53. The method of embodiment 52, wherein the rhenium-based catalyst is MTO.
54. The method of any one of embodiments 47 to 53, wherein the solvent system comprises an alcohol.
55. The method of embodiment 54, wherein the solvent system comprises ethanol.
56. The method of any one of embodiments 47 to 55, wherein the reaction mixture further comprises Pd/C.
57. The method of any one of embodiments 47 to 56, wherein the deoxydehydration reaction produces hex-2-enedioic acid diethyl ester.
58. The method of embodiment 57, wherein the method further comprises, reacting hex-2-enedioic acid diethyl ester with one or more reducing agents comprising hydrogen gas.
59. The method of embodiment 58, wherein the hydrogen gas is used at a pressure of up to 300 psi.
60. The method of embodiment 58, wherein hex-2-enedioic acid diethyl ester is reduced at a temperature greater than 20°C in a solvent system comprising a catalyst suitable for the hydrogenation of an alkene.
61. The method of embodiment 60, wherein the solvent system comprises an alcohol.
62. The method of embodiment 61, wherein the solvent system comprises ethanol.
63. The method of any one of embodiments 60 to 62, wherein the catalyst comprises Pd/C.
64. The method of embodiment 39, wherein the method further comprises the deoxydehydration/reduction of the lactone derivative using one or more reducing agents comprising hydrogen gas at a temperature from 220 to 295°C in a solvent system comprising (NH₄)₆Mo₇O₂₄.
65. The method of any one of embodiments 58 to 64, wherein the reduction reaction produces diethyl adipate.
66. A method for the deoxydehydration (DODH) of a sugar derivative, comprising:
   (a) incubating a reaction mixture at 220 to 295°C for a sufficient period of time to allow for formation of one or more deoxydehydrated products, wherein the reaction mixture comprises: a reactant having the structure of 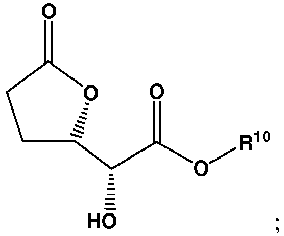 catalysts comprising (NH₄)₆Mo₇O₂₄ and Pd/C;
      a reducing agent comprising hydrogen gas;
      a solvent system comprising ethanol,
      wherein, R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
67. The method of embodiment 1, wherein the reaction mixture comprises a sugar lactone derivative having the structure of Formula V of Formula V(a): 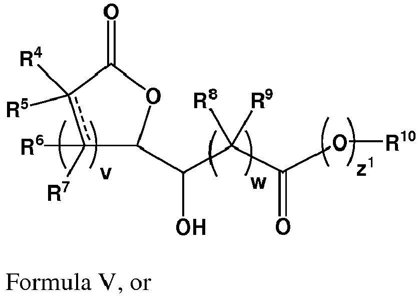 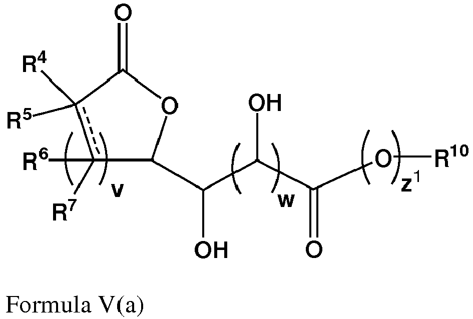 wherein,
   v is an integer selected from the group consisting of 1, 2, 3, 4, and 5;
   w is an integer selected from the group consisting of 0, 1, 2, 3, 4, 5 and 6;
   z¹ is an integer selected from 0 or 1;
   R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and
   R¹⁰ is selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
68. The method of embodiment 67, wherein the reaction is carried out by incubating the reaction mixture at a temperature greater than 20°C.
69. The method of embodiment 68, wherein the reaction mixture is incubated at a temperature between 120°C to 300°C.
70. The method of any one of embodiments 67 to 69, wherein the reaction is carried out for up to 72 hours.
71. The method of any one of embodiments 67 to 70, wherein the reaction mixture is incubated at about 150°C for up to 4 hours.
72. The method of any one of embodiments 67 to 71, wherein the catalyst is a rhenium-based catalyst.
73. The method of embodiment 72, where the rhenium based catalyst is methyltrioxorhenium (MTO).
74. The method of any one of embodiments 67 to 73, wherein the solvent system comprises an alcohol.
75. The method of embodiment 74, wherein the solvent system comprises ethanol.
76. The method of any one of embodiments 67 to 75, wherein the reaction mixture further comprises palladium on carbon (Pd/C).
77. The method of any one of embodiments 67 to 76, wherein the deoxydehydrated product of the deoxydehydration reaction comprises a structure of Formula VI or Formula VI(a): 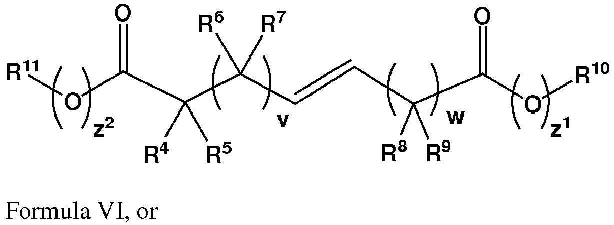 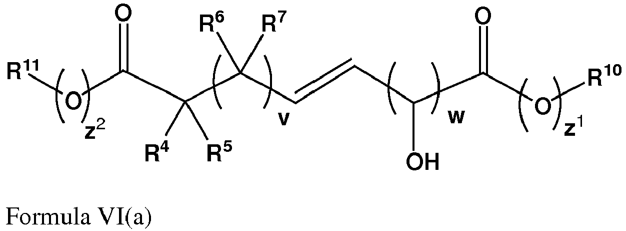 wherein,
   v is an integer selected from the group consisting of 1, 2, 3, 4, and 5;
   w is an integer selected from the group consisting of 0, 1, 2, 3, 4, 5 and 6;
   z¹ and z² are independently selected integers from 0 or 1;
   R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and
   R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
78. The method of embodiment 77, wherein the method further comprises, reducing the deoxydehydrated product using one or more reducing agents comprising hydrogen gas.
79. The method of embodiment 78, wherein the hydrogen gas is used at pressure of up to 300 psi.
80. The method of embodiment 78, wherein the deoxydehydrated product is reduced at a temperature greater than 20°C in a solvent system comprising a catalyst suitable for hydrogenating an alkene.
81. The method of embodiment 80, wherein the solvent system comprises an alcohol.
82. The method of embodiment 81, wherein the solvent system comprises ethanol.
83. The method of any one of embodiments 80 to 82, wherein the catalyst comprises Pd/C.
84. The method of any one of embodiments 78 to 83, wherein the reduction reaction produces a reduced product having a structure of Formula VII: 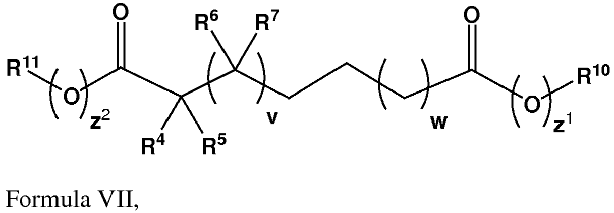 wherein,
   v is an integer selected from the group consisting of 1, 2, 3, 4, and 5;
   w is an integer selected from the group consisting of 0, 1, 2, 3, 4, 5 and 6;
   z¹ and z² are integers independently selected from 0 or 1;
   R⁴, R⁵, R⁶, R⁷, R⁸, and R⁹ are each independently an H, hydroxyl, halo, ester, alkoxy, alkenyloxy, thiol, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl; and
   R¹⁰ and R¹¹ are independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂)alkynyl, optionally substituted (C₂-C₁₁)heteroalkynyl, optionally substituted cycloalkyl, optionally substituted cycloalkenyl, optionally substituted heterocycle, and optionally substituted aryl.
85. A method for producing a compound having formula IV 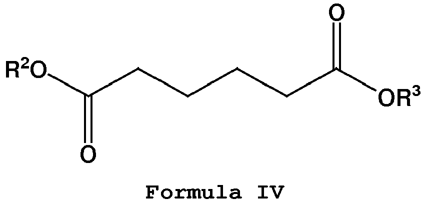 wherein R² and R³ are each independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl, from an C6-aldaric acid or C6-aldaric acid derivative, the method comprising reacting an C6-aldaric acid or C6-aldaric acid derivative in the presence of:
   (i) a catalyst selected from the group consisting of a vanadium-based catalyst, a molybdenum-based catalyst, a rhenium-based catalyst and any combination thereof;
   (ii) a further catalyst selected from the group consisting of a palladium-based catalyst and a platinum-based catalyst and any combination thereof;
   (iii) a reducing agent comprising hydrogen gas; and
   (iv) a solvent system.
86. The method of embodiment 85, wherein the C6 aldaric acid has the formula: HOOC-(CHOH)₄-COOH; and wherein the C6 aldaric acid derivative is a mono- or diester of C6 aldaric acid, a mono- or disalt of C6 aldaric acid, a dilactone of C6 aldaric acid, or a mono lactone of C6 aldaric acid.
87. The method of embodiment 86, wherein the aldaric acid is glucaric acid or galactaric acid.
88. The method of embodiment 86, wherein:
   the C6 aldaric acid derivative is a mono-or diester of glucaric acid or galactaric acid,
   the mono- or disalt of C6 aldaric acid is a mono-or disalt of glucaric acid or galactaric acid,
   the dilactone of C6 aldaric acid is a Glucaro-1,4:6,3-dilactone or an enantiomere or a diastereomere thereof,
   the mono lactone of C6 aldaric acid is a 6,3 glucarolactone or an enantiomere or a diastereomere thereof, or an ester of 6,3 glucarolactone or an enantiomere or a diastereomere thereof.
89. The method of embodiment 88, wherein the esters within the C6 aldaric acid derivatives are C1-C6-alkyl esters.
90. The method of embodiment 89, wherein the C1-C6-alkyl esters are methyl esters or ethyl esters.
91. The method of embodiment 85, wherein the reaction is carried out at a temperature greater than 20°C.
92. The method of embodiment 91, wherein the reaction is carried out at a temperature between 120°C and 300°C.
93. The method of embodiment 92, wherein the reaction is carried out at a temperature between 130 and 170°C.
94. The method of embodiment 85, wherein the solvent system comprises an nonpolar solvent, a polar organic solvent, an alcohol, water or a combination thereof.
95. The method of embodiment 94, wherein:
   the nonpolar solvent is selected from the group comprising toluene, benzene, xylene, hexane, diethyl ether, dichloromethane, and 1,4-dioxane;
   the polar organic solvent is selected from the group comprising tetrahydrofuran, ethyl acetate, acetone, dimethylformamide, acetonitrile, and dimethyl sulfoxide; and
   the alcohol is selected from the group comprising methanol, ethanol, isopropanol, n-propanol, and n-butanol.
96. The method of embodiment 94, wherein the solvent system comprises an alcohol.
97. The method of embodiment 96, wherein the solvent system comprises ethanol.
98. The method of embodiment 96, wherein the solvent system comprises methanol.
99. The method of embodiment 85, wherein the reaction is carried out in the presence of an acid.
100. The method of embodiment 99, wherein the acid is a Bronsted acid.
101. The method of embodiment 99, wherein the acid is an inorganic acid.
102. The method of embodiment 101, wherein the inorganic acid is selected from the group consisting of HCl, H₂SO₄, and H₃PO₄.
103. The method of embodiment 99, wherein the acid is an organic acid.
104. The method of embodiment 103, wherein the organic acid is selected from the group consisting of a C1-C3-alkane carboxylic acid, C1-C3-haloalkane carboxylic acids, C1-C3-alkane sulfonic acid, C1-C3-haloalkane sulfonic acid, and an optionally substituted benzene sulfonic acid.
105. The method of embodiment 103, wherein the organic acid is a C1-C3-alkane carboxylic acid or a C1-C3-haloalkane carboxylic acids.
106. The method of embodiment 105, wherein the organic acid is selected from acetic acid, chloroacetic acid, trichloroacetic acid, and trifluoroacetic acid.
107. The method of embodiment 103, wherein the organic acid is a C1-C3-alkane sulfonic acid, C1-C3-haloalkane sulfonic acid, or optionally substituted benzene sulfonic acid.
108. The method of embodiment 107, wherein the organic acid is a methansulfonic acid or trifluoromethansulfonic acid.
109. The method of embodiment 85, wherein the catalyst is selected from the group consisting of a vanadium-based catalyst, a molybdenum-based catalyst, a rhenium-based catalyst, and any combination thereof.
110. The method of embodiment 109, wherein the rhenium-based catalyst is selected from the group consisting of HReO₄, NaReO₄, KReO₄, NH₄ReO₄, ReO₂, ReIO₂(Ph₃P)₂, ReCl₃O(Ph₃P)₂, CH₃ReO₃ (MTO), and ReCl₃.
111. The method of embodiment 110, wherein the catalyst is MTO.
112. The method of embodiment 110, wherein the catalyst is HReO₄, NaReO₄, KReO₄, or NH₄ReO₄.
113. The method of embodiment 110, wherein the catalyst is NaReO₄ or KReO₄.
114. The method of embodiment 109, wherein the vanadium-based catalyst is selected from the group consisting of NBu₄VO₃, NBu₄VO₂(CA)₂, HC(PZ)VO₂BF₄, TpaVO₂PF₆, NaVO₂(acac)₂, and Bu₄N(dipic)VO₂.
115. The method of embodiment 109, wherein the molybdenum-based catalyst is selected from the group consisting of MoO₃, Mo(CO)₆, Mo(CO)₄(bipy), MoO₂Cl₂(bipy), MoO₂Br₂(bipy), MoO₂(CH3)₂(bipy),(NH₄)₆Mo₇O₂₄·4H₂O, and H₃PMo₁₂O₄₀.
116. The method of embodiment 85, wherein the further catalyst is selected from the group consisting of a palladium-based catalyst, a platinum-based catalyst, and any combination thereof.
117. The method of embodiment 116, wherein the palladium-based catalyst is a heterogeneous palladium-based catalyst.
118. The method of embodiment 116, wherein the platinum-based catalyst is a heterogeneous platinum-based catalyst.
119. The method of embodiment 117, wherein the heterogeneous palladium-based catalyst is palladium on a support, wherein the support is selected from the group consisting of Al₂O₃, ZrO₂, TiO₂, CeO₂, any combination thereof, and carbon.
120. The method of embodiment 119, wherein the heterogeneous palladium-based catalyst comprises palladium on carbon.
121. The method of embodiment 118, wherein the heterogeneous platinum-based catalyst is platinum on a support, wherein the support is selected from the group consisting of Al₂O₃, ZrO₂, TiO₂, CeO₂, any combination thereof, and carbon.
122. The method of embodiment 121, wherein the heterogeneous platinum-based catalyst is platinum on carbon.
123. The method of embodiment 85 or 109, wherein the reaction is carried out in the absence of an additional added acid.
124. The method of embodiment 85 or 109, wherein the reaction is carried out in the presence of an acid.
125. The method of embodiment 85, wherein hydrogen gas is used at a pressure from about 14.7 psi to about 500 psi.
126. The method of embodiment 123, wherein hydrogen gas is used at a pressure from about 14.7 psi to about 500 psi.
127. The method of embodiment 124, wherein the hydrogen gas is used at a pressure from about 14.7 psi to about 500 psi.
128. The method of embodiment 125, 126 or 127, wherein the hydrogen gas is used at a pressure from about 14.7 psi to about 30 psi.
129. The method of embodiment 85 or 110, wherein
   the catalyst selected from the group consisting of a vanadium-based catalyst, a molybdenum-based catalyst, a rhenium-based catalyst, and any combination thereof (catalyst 1), and
   the further catalyst selected from the group consisting of a palladium-based catalyst and a platinum-based catalyst, and any combination thereof (catalyst 2),
   are added in several portions during the reaction, and
   wherein the respective portion of the catalyst 1 and the respective portion of catalyst 2 are added at essentially the same point of time.
130. The method of embodiment 129,
   wherein the catalyst 1 and the catalyst 2 are added in several portions during the reaction, and
   wherein additionally a further catalyst selected from the group consisting of a palladium-based catalyst, a platinum-based catalyst, and any combination thereof (catalyst 3) is added in several portions during the reaction, and
   wherein the respective portion of the catalyst 1 and the respective portions of catalyst 2 are added at essentially the same point of time, and
   wherein the respective portion of catalyst 3 is added after the respective portions of the catalyst 1 and the catalyst 2.
131. The method of embodiment 130, wherein catalyst 2 and catalyst 3 are identical.
132. The method of embodiment 85, 109, or 123, wherein
   the catalyst is selected from the group consisting of a vanadium-based catalyst, a molybdenum-based catalyst, a rhenium-based catalyst and any combination thereof (catalyst 1), and
   the further catalyst is selected from the group consisting of a palladium-based catalyst and a platinum-based catalyst and any combination thereof (catalyst 2),
   wherein the catalyst 1 and catalyst 2 are added before the start of the reaction;
   a hydrogen gas is used at a pressure from about 14.6 psi to 30 psi; and
   wherein after at least partial formation of a compound of formula I a further catalyst selected from the group consisting of a palladium-based catalyst, a platinum-based catalyst, and any combination thereof (catalyst 3), is added and
   the hydrogen gas is used at a pressure from about 14.6 psi to 30 psi.
133. The method of embodiment 85, 109, or 123 wherein
   the catalyst is selected from the group consisting of a vanadium-based catalyst, a molybdenum-based catalyst, a rhenium-based catalyst and any combination thereof (catalyst 1), and
   the further catalyst is selected from the group consisting of a palladium-based catalyst and a platinum-based catalyst and any combination thereof (catalyst 2),
   wherein the catalyst 1 and the catalyst 2 are added before the start of the reaction;
   a hydrogen gas is used at a pressure from about 14.6 psi to 30 psi; and
   wherein after at least partial formation of a compound of formula I the hydrogen gas pressure is increased to about 50 to 400 psi.
134. The method of embodiment 133, wherein after at least partial formation of a compound of formula I the hydrogen gas pressure is increased to about 250 to 350 psi.
135. The method of embodiment 85, 109 or 123, wherein
   the catalyst is selected from the group consisting of a vanadium-based catalyst, a molybdenum-based catalyst, a rhenium-based catalyst, and any combination thereof (catalyst 1), and
   the further catalyst selected from the group consisting of a palladium-based catalyst, a platinum-based catalyst, and any combination thereof (catalyst 2),
   wherein a portion of the catalyst 1 and a portion of catalyst 2 are added before the start of the reaction;
   a hydrogen gas is used at a pressure from about 14.6 psi to 30 psi;
   wherein after at least partial formation of a compound of formula I, a further catalyst selected from the group consisting of a palladium-based catalyst and a platinum-based catalyst, and any combination thereof (catalyst 3) is added and the hydrogen gas is used at a pressure from about 14.6 psi to 30 psi;
   wherein after at least partial formation of a compound of formula II, a further portion of the catalyst 1 and a further portion of the catalyst 2, are added;
   the hydrogen gas is used at a pressure from about 14.6 psi to 30 psi; and
   after at least partial formation of a compound of formula III, a further portion of the catalyst 3 is added and the hydrogen gas is used at a pressure from about 14.6 psi to 30 psi.
136. The method of embodiment 135, wherein the catalyst 2 and the catalyst 3 are identical.
137. The method of embodiment 85, 109 or 123, wherein
   the catalyst is selected from the group consisting of a vanadium-based catalyst, a molybdenum-based catalyst, a rhenium-based catalyst, and any combination thereof (catalyst 1), and
   the further catalyst is selected from the group consisting of a palladium-based catalyst, a platinum-based catalyst, and any combination thereof (catalyst 2),
   wherein a portion of the catalyst 1 and a portion of the catalyst 2, are added before the start of the reaction;
   the hydrogen gas is used at a pressure from about 14.6 psi to about 30 psi;
   wherein after at least partial formation of a compound of formula I the hydrogen gas pressure is increased to about 50 to about 400 psi;
   wherein after at least partial formation of a compound of formula II a further portion of the catalyst 1 and a further portion of the catalyst 2, are added;
   the hydrogen gas is reduced to a pressure from about 14.6 psi to about 30 psi; and
   wherein after at least partial formation of a compound of formula III; the hydrogen gas is increased to about 50 to about 400 psi.
138. The method of embodiment 137, wherein after at least partial formation of a compound of formula I, the hydrogen gas pressure is increased to about 250 to about 350 psi, and wherein after at least partial formation of a compound of formula III, the hydrogen gas pressure is increased to about 250 to about 350 psi.
139. The method of embodiment 85, 109 or 123 wherein
   the catalyst is selected from the group consisting of a vanadium-based catalyst, a molybdenum-based catalyst, a rhenium-based catalyst, and any combination thereof (catalyst 1), and
   the further catalyst is selected from the group consisting of a palladium-based catalyst and a platinum-based catalyst and any combination thereof (catalyst 2),
   wherein the catalyst 1 and the catalyst 2, are added before the start of the reaction;
   the hydrogen gas is used at a pressure from about 14.6 psi to about 30 psi;
   the temperature is from about 130 to about 170°C;
   wherein after at least partial formation of a compound of formula I, the hydrogen gas pressure is increased to about 50 to about 400 psi;
   the temperature is from about 130 to about 170°C;
   wherein after at least partial formation of a compound of formula II the reaction mixture is treated with an oxygen comprising gas, in particular air or an oxygen gas, which is diluted with an inert gas, e.g. nitrogen or a noble gas, at a temperature from about 15 to about 50°C, in particular at room temperature; followed by reacting the reaction mixture first with hydrogen gas at a pressure from about 14.6 psi to about 30 psi and a temperature from about 130 to about 170°C and secondly with hydrogen gas at a pressure from about 50 to about 400 psi, in particular from about 250 to about 350 psi and a temperature from about 130 to about 170°C;
   and wherein optionally before the treatment with the oxygen comprising gas hydrogen is removed from the reaction mixture; and
   optionally the treatment with said oxygen comprising gas and the hydrogen gas is repeated at least once, in particular one or two times.
140. The method of embodiment 85, wherein the ratio of the catalyst to the further catalyst (mol:mol) is from 20:1 to 1:5,
141. The method of embodiment 140, wherein the ratio of the catalyst to the further catalyst (mol:mol) is from 10:1 to 1:1.
142. The method of embodiment 85, wherein the catalyst is selected from the group consisting of a vanadium-based catalyst, a molybdenum-based catalyst, a rhenium-based catalyst and any combination thereof; and wherein the reaction is carried out in the presence of an acid, in particular a Bronsted acid.
143. The method of embodiment 142, wherein the rhenium-based catalyst is selected from the group consisting of HReO₄, NaReO₄, KReO₄, NH₄ReO₄, ReO₂, ReIO₂(Ph₃P)₂, ReCl₃O(Ph₃P)₂, CH₃ReO₃ (MTO), and ReCl₃.
144. The method of embodiment 143, wherein the catalyst is HReO₄, NaReO₄, KReO₄ or NH₄ReO₄, and wherein the acid is an inorganic acid, in particular HCl, H₂SO₄, H₃PO₄.
145. The method of embodiment 144, wherein the catalyst is NaReO₄ or KReO₄; and wherein the acid is an inorganic acid, in particular HCl, H₂SO₄, H₃PO₄.
146. The method of embodiment 143, wherein the catalyst is NaReO₄ or KReO₄; and wherein the acid is H₃PO₄.
147. The method of embodiment 143, wherein the catalyst is HReO₄, NaReO₄, KReO₄ or NH₄ReO₄; and wherein the acid is an organic acid.
148. The method of embodiment 147, wherein the catalyst is NaReO₄ or KReO₄; and wherein the acid is an organic acid.
149. The method of embodiment 147 or 148, wherein the organic acid is selected from the group consisting of a C1-C3-alkane carboxylic acid, C1-C3-haloalkane carboxylic acid, C1-C3-alkane sulfonic acid, C1-C3-haloalkane sulfonic acid, and an optionally substituted benzene sulfonic acid.
150. The method of embodiment 143, wherein the catalyst is NaReO₄ or KReO₄; and wherein the acid is a C1-C3-alkane carboxylic acid or a C1-C3-haloalkane carboxylic acid.
151. The method of embodiment 150, wherein the acid is selected from the group consisting of acetic acid, chloroacetic acid, trichloroacetic acid, and trifluoroacetic acid.
152. The method of embodiment 143, wherein the catalyst is NaReO₄ or KReO₄; and wherein the acid is a C1-C3-alkane sulfonic acid, C1-C3-haloalkane sulfonic acid, or optionally substituted benzene sulfonic acid, in particular selected from methansulfonic acid or trifluoromethansulfonic acid.
153. The method of any one of embodiments 142 to 146, wherein the C6 aldaric acid is HOOC-(CHOH)₄-COOH, in particular glucaric acid or galactaric acid; and wherein the C6 aldaric acid derivative is a mono- or diester of C6 aldaric acid, in particular a mono-or diester of glucaric acid or galactaric acid, a mono- or disalt of C6 aldaric acid, in particular a mono-or disalt of glucaric acid or galactaric acid, a dilactone of C6 aldaric acid, in particular Glucaro-1,4:6,3-dilactone or an enantiomere or a diastereomere thereof; a mono lactone of C6 aldaric acid, in particular 6,3 glucarolactone or an enantiomere or a diastereomere thereof; or any combination thereof, in particular an ester of 6,3 glucarolactone or an enantiomere or a diastereomere thereof.
154. The method of embodiment 153, wherein the esters within the C6 aldaric acid derivatives are C₁-C₆-alkyl esters, in particular methyl esters or ethyl esters.
155. The method of embodiment 153 or embodiment 85, wherein the C6 aldaric acid or the C6 aldaric acid derivative is selected from the group consisting of glucaric acid, glucaric acid dimethyl ester, glucaric acid diethylester, Glucaro-1,4:6,3-dilactone or an enantiomere or a diastereomere thereof, 6,3 glucarolactone, 6,3 glucarolactone methyl ester, 6,3 glucarolactone ethyl ester and its salts, in particular selected from the group consisting of glucaric acid, glucaric acid dimethyl ester, glucaric acid diethylester, Glucaro-1,4:6,3-dilactone or an enantiomere or a diastereomere thereof, 6,3 glucarolactone or an enantiomere or a diastereomere thereof, 6,3 glucarolactone methyl ester or an enantiomere or a diastereomere thereof and 6,3 glucarolactone ethyl ester or an enantiomere or a diastereomere thereof.
156. The method of embodiment 153, wherein the further catalyst is selected from the group consisting of a palladium-based catalyst, a platinum-based catalyst and any combination thereof, and is a heterogeneous palladium-based catalyst, a heterogeneous platinum-based catalyst or any combination thereof.
157. The method of embodiment 156, wherein the heterogeneous palladium-based catalyst is palladium on a support, in particular wherein the support is selected from the group of Al₂O₃, ZrO₂, TiO₂, CeO₂, or any combination thereof, and carbon; in particular palladium on carbon.
158. The method of embodiment 156, wherein the heterogeneous platinum-based catalyst is platinum on a support, in particular wherein the support is selected from the group of Al₂O₃, ZrO₂, TiO₂, CeO₂, or any combination thereof, and carbon; in particular platinum on carbon.
159. The method of embodiment 142, wherein the reaction is carried out at a temperature greater than 20°C.
160. The method of embodiment 142, reaction is carried out at a temperature between 120°C and 300°C, in particular at a temperature between 130 and 170°C.
161. The method of embodiment 142, wherein the solvent system comprises a nonpolar solvent selected from the group consisting of toluene, benzene, xylene, hexane, diethyl ether, dichloromethane, or 1,4-dioxane; a polar organic solvent selected from the group consisting of tetrahydrofuran, ethyl acetate, acetone, dimethylformamide, acetonitrile, and dimethyl sulfoxide; an alcohol selected from the group consisting of particular methanol, ethanol, isopropanol, n-propanol, and n-butanol; water or a combination of any of the foregoing.
162. The method of embodiment 161, wherein the solvent system comprises an alcohol.
163. The method of embodiment 161, wherein the solvent system comprises ethanol.
164. The method of embodiment 161, wherein the solvent system comprises methanol.
165. The method of embodiment 142, wherein the hydrogen gas is used at a pressure from about 14.7 to about 200 psi, in particular from about 50 to 100 psi.
166. The method of embodiment 142, wherein the ratio of the catalyst to the further catalyst (mol:mol) are from 20:1 to 1:5, in particular from 5:1 to 1:1.
167. The method of embodiment 142 or 166,
   wherein the catalyst is NaReO₄ or KReO₄;
   wherein the further catalyst is a heterogeneous palladium-based catalyst, a heterogeneous platinum-based catalyst or any combination thereof;
   wherein the acid is H₃PO₄, acetic acid or trifluoroacetic acid;
   wherein the C6 aldaric acid is HOOC-(CHOH)₄-COOH, in particular glucaric acid, galactaric acid;
   wherein the C6 aldaric acid derivative is a mono- or diester of C6 aldaric acid, in particular a mono-or diester of glucaric acid or galactaric acid, a mono- or disalt of C6 aldaric acid, in particular a mono-or disalt of glucaric acid or galactaric acid, a dilactone of C6 aldaric acid, in particular Glucaro-1,4:6,3-dilactone or an enantiomere or a diastereomere thereof; a mono lactone of C6 aldaric acid, in particular 6,3 glucarolactone or an enantiomere or a diastereomere thereof; or any combination thereof, in particular an ester of 6,3 glucarolactone or an enantiomere or a diastereomere thereof;
   wherein reaction is carried out at a temperature between 120°C and 300°C, in particular at a temperature between 130 and 170°C;
   wherein the solvent system comprises an alcohol, in particular methanol or ethanol; and
   wherein the hydrogen gas is used at a pressure from about 14.7 to about 200 psi, in particular from about 50 to 100 psi.

A number of embodiments have been described herein. Nevertheless, it will be understood that various modifications may be made without departing from the spirit and scope of this disclosure. Accordingly, other embodiments are within the scope of the following claims.

## Claims

1. A method for producing a compound having formula IV 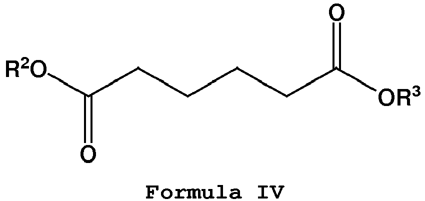 wherein R² and R³ are each independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl, from an C6-aldaric acid or C6-aldaric acid derivative, the method comprising reacting an C6-aldaric acid or C6-aldaric acid derivative in the presence of:
(i) a rhenium-based catalyst;
(ii) a further catalyst selected from the group consisting of a palladium-based catalyst and a platinum-based catalyst and any combination thereof;
(iii) a reducing agent comprising hydrogen gas; and
(iv) a solvent system.

2. The method of claim 1, wherein the C6 aldaric acid has the formula: HOOC-(CHOH)4-COOH; and wherein the C6 aldaric acid derivative is a mono- or diester of C6 aldaric acid, a mono- or disalt of C6 aldaric acid, a dilactone of C6 aldaric acid, or a mono lactone of C6 aldaric acid.

3. The method according to any preceding claim, wherein the reaction is carried out in the presence of an acid, in particular a Bronsted acid, wherein the rhenium-based catalyst is preferably selected from the group consisting of HReO4, NaReO4, KReO4, NH4ReO4, ReO2, ReIO2(Ph3P)2, ReCl3O(Ph3P)2, CH3ReO3 (MTO), and ReCl3.

4. The method according to any preceding claim, wherein wherein the reaction is carried out at a temperature between 120°C and 300°C, wherein the reaction is preferably carried out at a temperature between 130 and 170°C.

5. The method according to any preceding claim, wherein
the palladium-based catalyst is a heterogeneous palladium-based catalyst
and/or
the platinum-based catalyst is a heterogeneous platinum-based catalyst.

6. The method according to any preceding claim,
wherein the reaction is carried out in the presence of an acid, in particular a Bronsted acid,
wherein the catalyst is NaReO4 or KReO4;
wherein the further catalyst is a heterogeneous palladium-based catalyst, a heterogeneous platinum-based catalyst or any combination thereof;
wherein the acid is H3PO4, acetic acid or trifluoroacetic acid;
wherein the C6 aldaric acid is HOOC-(CHOH)4-COOH, in particular glucaric acid, galactaric acid;
wherein the C6 aldaric acid derivative is a mono- or diester of C6 aldaric acid, in particular a mono-or diester of glucaric acid or galactaric acid, a mono- or disalt of C6 aldaric acid, in particular a mono-or disalt of glucaric acid or galactaric acid, a dilactone of C6 aldaric acid, in particular Glucaro-1,4:6,3-dilactone or an enantiomere or a diastereomere thereof; a mono lactone of C6 aldaric acid, in particular 6,3 glucarolactone or an enantiomere or a diastereomere thereof; or any combination thereof, in particular an ester of 6,3 glucarolactone or an enantiomere or a diastereomere thereof;
wherein reaction is carried out at a temperature between 120°C and 300°C, in particular at a temperature between 130 and 170°C;
wherein the solvent system comprises an alcohol, in particular methanol or ethanol; and
wherein the hydrogen gas is used at a pressure from about 14.7 to about 200 psi, in particular from about 50 to 100 psi.

7. The method according to any preceding claim, for producing a compound having formula IV 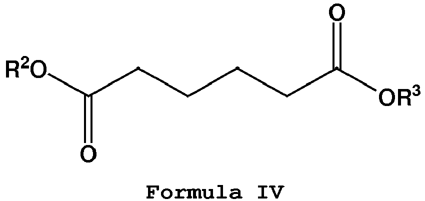 wherein R² and R³ are each independently selected from the group consisting of H, optionally substituted (C₁-C₁₂)alkyl, optionally substituted (C₁-C₁₁)heteroalkyl, optionally substituted (C₂-C₁₂)alkenyl, optionally substituted (C₂-C₁₁)heteroalkenyl, optionally substituted (C₂-C₁₂) alkynyl, optionally substituted (C₂-C₁₁)heteroalkynyl, optionally substituted cycloalkyl, optionally substituted cycloalkenyl, optionally substituted heterocycle, and optionally substituted aryl, from an C6-aldaric acid or C6-aldaric acid derivative, the method comprising reacting an C6-aldaric acid or C6-aldaric acid derivative in the presence of:
(i) a rhenium-based catalyst;
(ii) a further catalyst selected from the group consisting of a palladium-based catalyst and a platinum-based catalyst and any combination thereof;
(iii) a reducing agent comprising hydrogen gas; and
(iv) a solvent system,
wherein the reaction is carried out in the presence of an acid, .
the C6 aldaric acid is HOOC-(CHOH)₄-COOH,
the C6 aldaric acid derivative is a mono- or diester of C6 aldaric acid, a mono- or disalt of C6 aldaric acid, a dilactone of C6 aldaric acid or an enantiomere or diastereomere thereof, a mono lactone of C6 aldaric acid or an enantiomere or diastereomere thereof, or any combination thereof,
the reaction is carried out at a temperature between 120°C and 300°C;
the solvent system comprises an alcohol; and
the hydrogen gas is used at a pressure from about 14.7 to about 200 psi.

8. The method according to any preceding claim, wherein
the C6 aldaric acid is glucaric acid or galactaric acid; and
the C6 aldaric acid derivative is a mono-or diester of glucaric acid or galactaric acid, a mono-or disalt of glucaric acid or galactaric acid, Glucaro-1,4:6,3-dilactone or an enantiomere or a diastereomere thereof, 6,3 glucarolactone or an enantiomere or a diastereomere thereof, or any combination thereof.

9. The method according to any preceding claim, wherein the rhenium-based catalyst is HReO₄, NaReO₄, KReO₄, or NH₄ReO₄, preferably NaReO₄ or KReO₄.

10. The method according to any preceding claim, wherein the hydrogen gas is used at a pressure of about 50 to 100 psi.

11. The method according to any preceding claim, wherein the solvent system comprises an alcohol, preferably methanol or ethanol.

12. The method according to any preceding claim, wherein the reaction is conducted at a temperature between 130 and 170°C.

13. The method according to any preceding claim, wherein the acid is H₃PO₄, acetic acid, or trifluoroacetic acid.

14. The method according to any preceding claim, wherein the further catalyst is a heterogeneous palladium-based catalyst, a heterogeneous platinum-based catalyst, or a combination thereof.

15. The method according to any preceding claim, wherein the C6-aldaric acid derivative comprises an ester of 6,3 glucarolactone,
wherein preferably the ester comprises a C1-C6 alkyl ester,
wherein more preferably the ester is a methyl ester or an ethyl ester.

16. The method according to any preceding claim, wherein the further catalyst is palladium or platinum on a support selected from the group of Al₂O₃, ZrO₂, TiO₂, CeO₂, and carbon.

17. The method according to any preceding claim, wherein the further catalyst comprises palladium on carbon, platinum on carbon, or a mixture thereof.

18. The method according to any preceding claim, wherein
the rhenium-based catalyst and the further catalyst are added in several portions during the reaction, and wherein the respective portion of the rhenium-based catalyst and the respective portion of the further catalyst are added at essentially the same point of time.

19. The method according to any preceding claim, wherein a ratio of the rhenium-based catalyst to the further catalyst (mol:mol) is from 20:1 to 1:5,
wherein preferably a ratio of the rhenium-based catalyst to the further catalyst (mol:mol) is from 10:1 to 1:1.

20. The method according to any preceding claim, wherein
the catalyst is NaReO₄ or KReO₄;
the further catalyst is a heterogeneous palladium-based catalyst, a heterogeneous platinum-based catalyst, or a combination thereof;
the acid is H₃PO₄, acetic acid, or trifluoroacetic acid;
the C6 aldaric acid is glucaric acid or galactaric acid;
the C6 aldaric acid derivative is a mono-or diester of glucaric acid or galactaric acid, a mono-or disalt of glucaric acid or galactaric acid, a Glucaro-1,4:6,3-dilactone or an enantiomere or diastereomere thereof, or any combination thereof,
wherein the reaction is carried out at a temperature between 130 and 170°C,
the solvent system comprises methanol or ethanol, and
the hydrogen gas is used at a pressure from about 14.7 to about 200 psi.

21. The method according to claim 14, wherein the C6 aldaric acid derivative comprises an ester of 6,3 glucarolactone or an enantiomere or diastereomere thereof.
